# EUROPEAN PATENT APPLICATION

(11) **EP 4 667 562 A1**
(43) Date of publication of application: **24.12.2025**
(21) Application number: 24756786.0
(22) Date of filing: 08.02.2024
(51) Int. Cl.: C12N 5/071

(54) **THREE-DIMENSIONAL HEPATOCYTE TISSUE AND METHOD FOR PRODUCING SAME**

(30) Priority: 14.02.2023 JP 2023021079
(71) Applicant: Toppan Holdings Inc., Tokyo 110-0016 (JP)
(72) Inventor: HIRAOKA, Yasuyuki, Tokyo 110-0016 (JP)
(74) Representative: Plasseraud IP
(86) International application number: PCT/JP2024/004299
(87) International publication number: WO 2024/171941

(57) **Abstract**

The present invention relates to a three-dimensional hepatic tissue comprising a cell structure containing at least hepatocytes, and a hydrogel, wherein the cell structure does not contain hepatic stellate cells, and the cell structure is embedded in the hydrogel.

## Description

### [Technical Field]

The present invention relates to a three-dimensional hepatic tissue and a method of manufacturing the same.

### [Background Art]

In recent years, the advantages of three-dimensional cellular tissues, which are three-dimensionally organized cells, over cells grown on a flat plate, have been demonstrated not only in regenerative medicine but also in drug assay systems that require an environment that resembles the living body. Various techniques have been developed to produce three-dimensional cellular tissues in vitro. Examples of methods that have been developed include forming cell clusters on a surface substrate to which cells cannot attach, forming cell clusters in droplets, and accumulating cells on a permeable membrane. In order to maintain cellular organization, an extracellular matrix (ECM) such as collagen produced by the living body itself is required for intercellular binding and scaffold formation. To achieve this, adding an ECM from the outside to artificially produce cellular tissue has been considered. For example, PTL 1 discloses a cell culture method in which isolated cells are first brought into contact with one or more types of water-soluble polymeric substances (such as collagen) in an aqueous solution that have a cell protective effect. Then, the cells are allowed to form a three-dimensional aggregate and cultured on a permeable substrate floating on a medium. PTL 2 discloses a process for producing a three-dimensional structure of myocardium-like cells, which comprises culturing cells on a cell culture support having a substrate surface coated with a temperature-responsive polymer whose upper or lower critical solution temperature in water is 0 to 80°C, and subsequently: (1) bringing the temperature of the culture solution to above the upper critical solution temperature or below the lower critical solution temperature, and optionally (2) bringing the cultured cell sheet into close contact with a polymer membrane and (3) peeling the cell sheet off together with the polymer membrane. PTL 2 also discloses a process for producing a three-dimensional structure of myocardium-like cells, in which the myocardium-like cell sheet obtained using the above-described process is again allowed to adhere to a cell culture support, a cell culture support coated with a temperature-responsive polymer, a polymer membrane or a cellular sheet and a plurality of the assemblies are piled up. PTL 3 discloses a method of producing a three-dimensional cellular tissue, comprising: a step A of obtaining a mixture in which cells are suspended in a solution containing at least a cationic buffer solution, an extracellular matrix component, and a polymeric electrolyte; a step B of gathering the cells from the obtained mixture to form a cell aggregate on a substrate; and a step C of culturing the cells to obtain a three-dimensional cellular tissue. The step C is carried out after the steps A and B have been carried out at least once, the extracellular matrix component is collagen, the polymeric electrolyte is selected from the group consisting of glycosaminoglycan, dextran sulfate, rhamnan sulfate, fucoidan, carrageenan, polystyrene sulfonic acid, polyacrylamide-2-methylpropanesulfonic acid, polyacrylic acid, and combinations thereof, the concentration of the extracellular matrix component in the mixture is from 0.05 mg/mL or more and less than 1.0 mg/mL, and the concentration of the polymeric electrolyte in the mixture is 0.05 mg/mL or more and less than 1.0 mg/mL.

It is also well known that, in general, when culturing cells, cell function can be improved by culturing the cells in a system in which a plurality of types of cells coexist so as to partially mimic the in vivo composition, rather than culturing the cells alone. For example, it is known that the function of hepatocytes can be enhanced by co-culturing them with other non-parenchymal cells such as fibroblasts, sinusoidal endothelial cells, and hepatic stellate cells, rather than culturing them alone (e.g., NPL 1 and NPL 2).

### [Citation List]

### [Patent Literature]

PTL 1: JP 2824081 B
PTL 2: WO 2002/008387 A
PTL 3: JP 6427836 B

### [Non Patent Literature]

NPL 1: Ahmed et al., "3D liver membrane system by co-culturing human hepatocytes, sinusoidal endothelial and stellate cells", Biofabrication, 2017, May 26;9(2):025022.
NPL 2: Kulka et al., "Micro scale Collagen and Fibroblast Interactions Enhance Primary Human Hepatocyte Functions in Three-Dimensional Models", Gene Expr, 2020, Jun 12;20(1):1-18.

### [Summary of the Invention]

### [Problem to be Solved by the Invention]

Hepatocytes, parenchymal cells that form the liver, are highly differentiated cells in vivo and have unique functions such as drug metabolism. On the other hand, when hepatocytes are extracted from the living body and cultured in vitro, they are known to rapidly dedifferentiate and lose their unique functions if cultured in a conventional two-dimensional environment. It is also known that three-dimensional culture can somewhat suppress this dedifferentiation and allows hepatocytes to be cultured with their functions maintained to a certain degree. However, there are still cases such as the expression of some important metabolic enzymes not being sufficiently maintained and the production of bile canalicular structures being insufficient, for which further improvements are needed.

An object of the present invention is to provide a more functional three-dimensional hepatic tissue in which the expression of metabolic enzymes specific to hepatocytes is sufficiently maintained and functional structures such as bile canaliculi are sufficiently produced. Another object of the present invention is to provide a method of producing the three-dimensional hepatic tissue.

### [Solution to Problem]

The inventors have found that a more functional three-dimensional hepatic tissue, which could not be obtained with existing three-dimensional culture methods, can be produced by aggregating and organizing hepatocytes inside a hydrogel. In addition, the inventors have surprisingly found that the presence of some non-parenchymal cells, which was believed to improve liver function when co-cultured with hepatocytes in conventional culture systems, is actually detrimental in the production of highly functional three-dimensional hepatic tissue, thereby completing the present invention.

That is, the present invention encompasses the following inventions.
[1] A three-dimensional hepatic tissue including a cell structure containing at least hepatocytes, and a hydrogel, wherein
   the cell structure does not contain hepatic stellate cells, and
   the cell structure is embedded in the hydrogel.
[2] The three-dimensional hepatic tissue according to [1], wherein an exterior of the cell structure is covered with the hydrogel, and no hydrogel is present in intercellular spaces inside the cell structure.
[3] The three-dimensional hepatic tissue according to [1] or [2], wherein the cell structure includes bile canaliculi.
[4] The three-dimensional hepatic tissue according to any one of [1] to [3], wherein expression levels of CYP3A4 gene and CYP2C9 gene are maintained.
[5] The three-dimensional hepatic tissue according to any one of [1] to [4], wherein the hydrogel is a fibrin gel.
[6] The three-dimensional hepatic tissue according to any one of [1] to [5], further including an extracellular matrix component.
[7] The three-dimensional hepatic tissue according to [6], wherein the extracellular matrix component is a collagen component.
[8] The three-dimensional hepatic tissue according to any one of [1] to [7], further including a polyelectrolyte.
[9] The three-dimensional hepatic tissue according to [8], wherein the polyelectrolyte is heparin.
[10] A method of manufacturing three-dimensional hepatic tissue, the method including:
   causing a composition containing cells and a hydrogel-forming substance to gel to embed the cells in the hydrogel; and
   culturing the cells embedded in the hydrogel, wherein
   the cells contain hepatocytes and no hepatic stellate cells.
[11] The manufacturing method according to [10], wherein the composition further contains an extracellular matrix component.
[12] The manufacturing method according to [11], wherein the extracellular matrix component is a collagen component.
[13] The manufacturing method according to any one of [10] to [12], wherein the composition further contains a polyelectrolyte.
[14] The manufacturing method according to [13], wherein the polyelectrolyte is heparin.
[15] The manufacturing method according to any one of [10] to [14], wherein the hydrogel-forming substance includes fibrinogen and thrombin.

### [Advantageous Effects of the Invention]

According to the present invention, it is possible to provide a more functional three-dimensional hepatic tissue in which the expression of metabolic enzymes specific to hepatocytes is sufficiently maintained and functional structures such as bile canaliculi are sufficiently produced. Further, according to the present invention, a method of producing the three-dimensional hepatic tissue can also be provided.

### [Brief Description of the Drawings]

Fig. 1 is a graph showing the results of analyzing the expression levels of the CYP3A4 gene (A) and CYP2C9 gene (B) in Test Example 1.
Fig. 2 is a diagram showing the results of the principal component analysis in Test Example 2.
Fig. 3 is a graph showing the results of analyzing the expression level of the CYP3A4 gene in Test Example 2.
Fig. 4 is a photograph showing the results of observation using a confocal microscope in Test Example 3.
Fig. 5 is a graph comparing the occurrence rates of linear fluorescein signals of different lengths, as shown in Fig. 4, according to Test Example 3.
Fig. 6 is a graph showing the results of quantifying lactate dehydrogenase (LDH) in the culture supernatant in Test Example 4.
Fig. 7 is a graph showing the results of measuring the cell viability in Test Example 4.
Fig. 8 is a graph showing the results of measuring the cell viability after exposure to cyclosporine A (A) and atorvastatin (B) in Test Example 5.
Fig. 9 is a graph showing the results of measuring the cell viability after exposure to clomipramine (A) and simvastatin (B) in Test Example 5.
Fig. 10 is a photograph showing the results of observation using a confocal microscope in Test Example 7.
Fig. 11 is a graph comparing the total bile canaliculus length per tissue area based on the linear fluorescein signals in Fig. 10, according to Test Example 7.
Fig. 12 is a graph showing the results of evaluation of the amount of albumin secreted in Test Example 8.
Fig. 13 shows an image of the three-dimensional hepatic tissue in which viable cells are stained with Calcein-AM in Test Example 9.

### [Description of the Embodiments]

Embodiments of the present invention will be described in detail below. Note that the present invention is not limited to the following embodiments.

### [Three-dimensional hepatic tissue]

The term "cell structure" as used herein refers to an aggregate of cells (a cluster of cells) artificially produced by cell culture so that cells are arranged three-dimensionally. The cell structure may contain one or more types of cells.

The three-dimensional hepatic tissue according to this embodiment includes a cell structure containing at least hepatocytes and a hydrogel in which the cell structure is embedded. The cell structure being embedded in a hydrogel means that hydrogel is partially or completely covering the exterior of the cell structure, or partially or completely covering the exterior of the cell structure and partially or completely filling the intercellular gaps inside. In the three-dimensional hepatic tissue according to this embodiment, since the cell structure is embedded in a hydrogel, the three-dimensional arrangement is maintained and the three-dimensional structure can be maintained. Further, the three-dimensional hepatic tissue according to this embodiment has a cell structure that does not contain hepatic stellate cells.

Since the three-dimensional hepatic tissue according to this embodiment has the above-described configuration, the expression of metabolic enzymes specific to hepatocytes is sufficiently maintained, and functional structures such as bile canaliculi are adequately produced, which allows it to be used as a tissue that resembles liver tissue (liver model).

A three-dimensional hepatic tissue according to an embodiment can have a configuration in which the exterior of the cell structure is covered with hydrogel, the interior of the cell structure is densely packed with cells, and no hydrogel is present in the intercellular gaps inside the cell structure. Covering only the exterior with hydrogel allows the three-dimensional arrangement to be maintained, and thus the three-dimensional structure to be maintained, while the cells inside adhere to each other so that bile canaliculi are densely packed.

The shape of the three-dimensional hepatic tissue according to this embodiment is not particularly limited. For example, it may be a sphere, approximately a sphere, an ellipsoid, approximately an ellipsoid, a hemisphere, approximately a hemisphere, a semicircle, approximately a semicircle, a rectangular prism, or approximately a rectangular prism. Biological tissue includes sweat glands, lymphatic vessels, sebaceous glands, and the like, and has a more complex structure than three-dimensional hepatic tissue. Therefore, three-dimensional hepatic tissue can be easily distinguished from biological tissue. Further, the three-dimensional hepatic tissue may be an aggregate attached to a support or may be an aggregate not attached to a support. When the three-dimensional hepatic tissue is an aggregate attached to a support, the cells are aggregated in an approximately hemispherical (approximately dome-shaped) form on the support, which facilitates microscopic observation from above the support. In addition, because the three-dimensional hepatic tissue is approximately dome-shaped, the maximum thickness of the three-dimensional hepatic tissue per cell quantity can be kept relatively small compared to three-dimensional hepatic tissue formed by suspension culture while maintaining a three-dimensional environment. This facilitates observing the deep part of the three-dimensional hepatic tissue and supply of nutrients.

The total number of cells forming the cell structure according to this embodiment is not particularly limited, and may be determined as appropriate taking into consideration factors such as the thickness and shape of the three-dimensional hepatic tissue to be produced, and the size of the cell culture vessel to be used for its production. The total number of cells forming the cell structure according to this embodiment is also synonymous with the total number of cells forming the three-dimensional hepatic tissue according to this embodiment.

The cell structure according to this embodiment contains at least hepatocytes but does not contain hepatic stellate cells.

Hepatocytes are parenchymal cells of the liver and have functions such as secreting bile and plasma proteins. The hepatocytes that form the three-dimensional hepatic tissue may be primary hepatocytes taken from an animal's liver, cells obtained by culturing primary hepatocytes, a cultured cell line established from primary hepatocytes, or hepatoblasts artificially differentiated from stem cells. Examples of primary hepatocytes include primary human hepatocytes such as PXB cells. Examples of cultured cell lines include cell lines derived from inactivated hepatoma cells, such as HepG2. Examples of stem cells differentiated into hepatoblasts include embryonic stem cells (ES cells), induced pluripotent stem cells (iPS cells), and mesenchymal stem cells. The hepatocytes according to this embodiment are preferably non-cancerous cells such as primary hepatocytes and hepatoblasts, and more preferably PXB cells due to their ease of handling.

The number of types of hepatocytes contained in the cell structure according to the present embodiment may be one, or may be two or more. For example, the cell structure according to this embodiment may contain a plurality of hepatocytes having different genotypes of proteins that are involved in liver function. Conversely, all of the hepatocytes contained in the cell structure according to this embodiment may have the same genotype for proteins involved in liver function. Proteins involved in liver function include, for example, drug metabolizing enzymes.

The ratio (X1/X0 × 100) of the number of hepatocytes (X1) to the total number of cells (X0) in the cell structure of this embodiment may be 5% or more, preferably 10% or more, more preferably 15% or more, even more preferably 20% or more, still even more preferably 25% or more, still even more preferably 30% or more, still even more preferably 35% or more, still even more preferably 40% or more, still even more preferably 45% or more, still even more preferably 50% or more, still even more preferably 55% or more, still even more preferably 60% or more, and still even more preferably 65% or more, and may be 95% or less, preferably 90% or less, more preferably 80% or less, and even more preferably 75% or less. In order to obtain a tissue that resembles liver tissue even better, the ratio (X1/X0 × 100) of the number of hepatocytes (X1) to the total number of cells (X0) in the cell structure of this embodiment may be 60% or more and 80% or less, and preferably 60% or more and 70% or less.

Hepatic stellate cells are non-parenchymal liver cells (cells other than hepatocytes among the cells that make up the liver) that have functions such as storing vitamin A and are present in the space of Disse, the region between hepatocytes and the sinusoids in the liver. In conventional culture systems, it was considered that hepatic stellate cells improve liver function when co-cultured with hepatocytes. On the other hand, as shown in the examples described below, it has been found that the inclusion of hepatic stellate cells has adverse effects, and the expression level of metabolic enzymes specific to hepatocytes decreases significantly as the culture proceeds. That is, the three-dimensional hepatic tissue according to this embodiment sufficiently maintains the expression of metabolic enzymes specific to hepatocytes by not including hepatic stellate cells. It is believed that this is the reason the three-dimensional hepatic tissue according to this embodiment adequately maintains the metabolic functions of the liver.

The metabolic enzyme whose expression is maintained in the three-dimensional hepatic tissue of this embodiment may be, for example, a metabolic enzyme gene belonging to the cytochrome P450 superfamily, a metabolic enzyme gene associated with the phase II reaction in drug metabolism, or a transporter gene responsible for transporting substances in and out of hepatocytes. Examples of metabolic enzyme genes belonging to the cytochrome P450 superfamily include CYP3A4, CYP2C9, CYP1A1, CYP1A2, CYP2E1, and CYP27A1 genes. Since the CYP3A4 gene is an enzyme gene involved in the metabolism of many compounds, it is particularly important that its expression is maintained during the culture period. The expression levels of the CYP3A4 gene and the CYP2C9 gene are particularly sufficiently maintained in the three-dimensional hepatic tissue according to this embodiment.

As used herein, "expression of a metabolic enzyme gene is maintained" means that the expression level of the gene may be greater than or equal to 1/16, preferably greater than or equal to 1/8, more preferably greater than or equal to 1/4, and even more preferably greater than or equal to 1/2 of the expression level of the gene immediately after production of the three-dimensional hepatic tissue (e.g., one day after culture is initiated by embedding the cells in a hydrogel), and may be smaller than or equal to 8 times, preferably smaller than or equal to 4 times, and more preferably smaller than or equal to twice that expression level.

Here, the expression level of a gene refers to the amount of an expression product of the gene. The expression product may be mRNA, which is a transcription product of the gene, or a protein, which is a translation product thereof.

The amount of mRNA can be measured by, for example, quantitative RT-PCR, quantitative real-time RT-PCR, or quantitative Northern blotting. The amount of protein can be measured by Western blotting, ELISA, or the like.

In the three-dimensional hepatic tissue according to this embodiment, the cell structure may contain cells other than hepatocytes as long as they do not impair the effects of the present invention. The other cells may be, for example, mature somatic cells or undifferentiated cells such as stem cells. Specific examples of somatic cells include neuronal cells, dendritic cells, immune cells, vascular endothelial cells, lymphatic endothelial cells, fibroblasts, epithelial cells (excluding hepatocytes), cardiac muscle cells, islet cells, smooth muscle cells, smooth muscle cells, bone cells, alveolar epithelial cells, and spleen cells. Examples of stem cells include ES cells, iPS cells, and mesenchymal stem cells. The other cells may be normal cells, or cells whose cellular function is enhanced or suppressed, such as cancer cells. "Cancer cells" are cells that are derived from somatic cells and have acquired infinite proliferation potential.

The cell structure may particularly contain vascular endothelial cells in order to form a liver model that is even closer to the in vivo state. Vascular endothelial cells refer to flattened cells that form the surface of the lumen of blood vessels. When the cell structure includes vascular endothelial cells, the vascular endothelial cells may be, for example, sinusoidal endothelial cells or human umbilical vein-derived endothelial cells (HUVECs). Sinusoidal endothelial cells are non-parenchymal liver cells (cells that make up the liver other than hepatocytes) that have a characteristic morphology that differs from other vascular endothelial cells, such as having numerous clusters of small pores in their cytoplasm (cribrosa structure) and lacking a basement membrane. The vascular endothelial cells included in the three-dimensional hepatic tissue may be primary cells (primary vascular endothelial cells) taken from an animal liver (e.g., human liver), cells obtained by culturing primary cells, a cultured cell line established from primary cells, or cells artificially differentiated from stem cells. An example of the primary vascular endothelial cells is primary sinusoidal endothelial cells such as product number 5000 from Sciencell. An example of the cultured cell line is the cultured cell line with product number T0056 from Applied Biological Materials. Examples of stem cells to be differentiated include embryonic stem cells (ES cells) and induced pluripotent stem cells (iPS cells). The vascular endothelial cells contained in the cell structure according to this embodiment may be non-cancerous cells.

The ratio (X2/X0 × 100) of the number of vascular endothelial cells (X2) to the total number of cells (X0) in the cell structure may be 5% or more, preferably 10% or more, more preferably 12% or more, even more preferably 14% or more, still even more preferably 15% or more, still even more preferably 20% or more, and still even more preferably 25% or more, and may be 50% or less, preferably 45% or less, more preferably 40% or less, even more preferably 35% or less, still even more preferably 30% or less, still even more preferably 25% or less, still even more preferably 20% or less, and still even more preferably 18% or less. In order to obtain a tissue that resembles liver tissue even better, the ratio (X2/X0 × 100) of the number of vascular endothelial cells (X2) to the total number of cells (X0) in the cell structure may be 5% or more and 40% or less, preferably 5% or more and 35% or less, more preferably 10% or more and 35% or less, even more preferably 10% or more and 25% or less, and still even more preferably 12% or more and 20% or less. The number of sinusoidal endothelial cells relative to the total number of cells in the cell structure may fall within the above ranges.

The origin of the hepatocytes or other cells contained in the cell structure is not particularly limited. For example, they may be cells derived from mammals such as humans, monkeys, dogs, cats, rabbits, pigs, cows, mice, or rats.

The term "hydrogel" as used herein refers to a polymer that has been crosslinked by hydrogen bonds, ionic bonds, coordinate bonds, covalent bonds, or the like to form a three-dimensional network structure, and that contains a liquid such as water in the three-dimensional network structure. Examples of hydrogels include, but are not limited to, fibrin gel, collagen gel, gelatin gel, hyaluronic acid gel, alginate gel, and pectin gel. The hydrogel is preferably fibrin gel. These hydrogels may be used singly or in combination of two or more.

The three-dimensional hepatic tissue according to this embodiment may contain an extracellular matrix component. The extracellular matrix component may be located in at least part of the spaces between cells.

As used herein, the term "extracellular matrix component" refers to an aggregate of extracellular matrix molecules formed by a plurality of extracellular matrix molecules. An extracellular matrix molecule refers to a substance that exists outside the cells in an organism. The extracellular matrix can be any substance as long as it does not adversely affect cell growth and cell aggregate formation. Specific examples of the extracellular matrix molecules include, but are not limited to, collagen, elastin, proteoglycans, fibronectin, hyaluronic acid, laminin, vitronectin, tenascin, entactin, fibrillin, and cadherin. The extracellular matrix components may be used singly or in combination.

The extracellular matrix may be a modification or variant of the above extracellular matrix, or may be a polypeptide such as a chemically synthesized peptide, as long as it does not adversely affect cell growth and cell aggregate formation. The extracellular matrix may have repeats of the sequence represented by Gly-X-Y, which is characteristic of collagen. Gly represents a glycine residue, and X and Y each independently represent an amino acid residue. The plurality of Gly-X-Y sequences may be the same or different. By having repeats of the sequence represented by Gly-X-Y, restrictions on the arrangement of the molecular chain are reduced, and therefore, for example, the function of the extracellular matrix as a scaffold material for cell culture is improved. In an extracellular matrix having repeats of the sequence represented by Gly-X-Y, the proportion of the sequence represented by Gly-X-Y in the entire amino acid sequence may be 80% or more, and preferably 95% or more. The extracellular matrix may also be a polypeptide having an RGD sequence. The RGD sequence refers to a sequence represented by Arg-Gly-Asp (arginine residue-glycine residue-aspartic acid residue). The inclusion of the RGD sequence further promotes cell adhesion, which, for example, makes the extracellular matrix more suitable as a scaffold material for cell culture. Examples of extracellular matrices containing a sequence represented by Gly-X-Y and an RGD sequence include collagen, fibronectin, vitronectin, laminin, and cadherin.

Examples of collagen include fibrillar collagen and non-fibrillar collagen. Fibrillar collagen refers to collagen that is the main component of collagen fibers, and specific examples include type I collagen, type II collagen, and type III collagen. An example of non-fibrillar collagen is type IV collagen.

Examples of the proteoglycan include, but are not limited to, chondroitin sulfate proteoglycan, heparan sulfate proteoglycan, keratan sulfate proteoglycan, and dermatan sulfate proteoglycan.

The shape of the extracellular matrix component may be, for example, fibrous. Fibrous refers to a shape composed of a thread-like extracellular matrix component, or a shape composed of crosslinked molecules of a thread-like extracellular matrix component. At least a portion of the extracellular matrix components may be fibrous. The shape of an extracellular matrix component is the shape of a single clump of the extracellular matrix component (an extracellular matrix component aggregate) observed under a microscope, and the extracellular matrix component preferably has a mean diameter and/or mean length as described below. A fibrous extracellular matrix component includes thin threads (fibrils) formed by the assembly of a plurality of thread-like extracellular matrix molecules, threads formed by the further assembly of fibrils, and defibrillated versions of these threads. When the extracellular matrix includes an extracellular matrix component having a fibrous shape, the RGD sequence is preserved without being destroyed in the fibrous extracellular matrix component, and therefore it can function even more effectively as a scaffold for cell adhesion.

The extracellular matrix component may contain at least one selected from the group consisting of collagen, laminin, and fibronectin, and preferably contains collagen. The collagen is preferably fibrillar collagen, more preferably type I collagen. The fibrillar collagen may be a commercially available collagen, and a specific example thereof is type I collagen derived from pig skin, manufactured by NH Foods Ltd.

The extracellular matrix component may be an extracellular matrix component derived from an animal. Examples of animal species from which the extracellular matrix component may be derived include, but are not limited to, humans, pigs, and cows. Extracellular matrix components may be derived from one type of animal, or may be a combination of components derived from a plurality of types of animals.

The extracellular matrix component may include a fragmented extracellular matrix component. "Fragmented" means that an aggregate of the extracellular matrix component is broken down into smaller sizes. The fragmented extracellular matrix component may include a defibrillated extracellular matrix component. A defibrated extracellular matrix component is a component obtained by defibrating the above extracellular matrix component by applying a physical force. For example, defibrillation is performed under conditions that do not cleave bonds within the extracellular matrix molecules.

A fragmented extracellular matrix component can be produced, for example, by a method including fragmenting an extracellular matrix component (fragmentation step).

The method of fragmenting the extracellular matrix component is not particularly limited, and it may be fragmented by applying a physical force. Unlike enzyme treatment, the molecular structure of the extracellular matrix component fragmented by application of a physical force usually does not change from that before fragmentation (the molecular structure is maintained). The extracellular matrix component may also be fragmented, for example, by breaking down a clump of extracellular matrix component into fragments. The extracellular matrix component may be fragmented in the solid phase or in an aqueous medium. For example, the extracellular matrix component may be fragmented by application of physical force using an ultrasonic homogenizer, a stirring homogenizer, or a high-pressure homogenizer. When a stirring homogenizer is used, the extracellular matrix component may be homogenized as it is, or may be homogenized in an aqueous medium such as physiological saline. Millimeter- or nanometer-sized fragments of the extracellular matrix component can be obtained by adjusting the homogenization time, number of times of homogenization, or the like. When the extracellular matrix component is fragmented in an aqueous medium, the fragmented extracellular matrix component can be produced, for example, by a method including fragmenting the extracellular matrix component in the aqueous medium, and removing the aqueous medium from a liquid containing the fragmented extracellular matrix component and the aqueous medium (removal step). The removal step may be carried out, for example, by freeze-drying. "Removing the aqueous medium" does not mean that no moisture is attached to the fragmented extracellular matrix component, but that water is removed to a degree that can reasonably be achieved by the general drying technique described above.

The diameter and length of the fragmented extracellular matrix component can be determined by analyzing individual fragments of the extracellular matrix component with an electron microscope.

The mean length of the fragments of the extracellular matrix component may be 100 nm or more and 400 µm or less, and preferably 100 nm or more and 200 µm or less. In an embodiment, in order to facilitate the formation of a thick three-dimensional hepatic tissue, the mean length of the fragments of the extracellular matrix component may be 5 µm or more and 400 µm or less, preferably 10 µm or more and 400 µm or less, and more preferably 100 µm or more and 400 µm or less. In another embodiment, the mean length of the fragments of the extracellular matrix component may be 100 µm or less, preferably 50 µm or less, more preferably 30 µm or less, even more preferably 15 µm or less, still even more preferably 10 µm or less, and still even more preferably 1 µm or less, and 100 nm or more. It is preferable that the mean length of the majority of the fragments of the extracellular matrix component is within the above numerical range. Specifically, it is preferable that the mean length of 50% or more of all the fragments of the extracellular matrix component is within the above numerical range, and it is more preferable that the mean length of 95% of the fragments of the extracellular matrix component is within the above numerical range. The fragmented extracellular matrix component is preferably a collagen component that has been fragmented so as to have a mean length within the above range.

The mean diameter of the fragments of the extracellular matrix component may be in the range of 50 nm to 30 µm, preferably 4 µm to 30 µm, and more preferably 5 µm to 30 µm. The fragmented extracellular matrix component is preferably a collagen component that has been fragmented so as to have a mean diameter within this range.

The mean diameter and mean length of the fragmented extracellular matrix component can be determined by measuring individual fragments of the extracellular matrix component with an optical microscope or the like, and performing image analysis. As used herein, "mean length" refers to the mean length of the measured sample in the longitudinal direction, and "mean diameter" refers to the mean length of the measured sample in the direction perpendicular to the longitudinal direction.

A collagen component that has been fragmented is also referred to as a "fragmented collagen component". The term "fragmented collagen component" refers to a collagen component, such as a fibrous collagen component, that has been fragmented but maintains its triple helix structure. The mean length of the fragments of the collagen components is preferably in the range of 100 nm to 200 µm, more preferably 22 µm to 200 µm, and even more preferably 100 µm to 200 µm. The mean diameter of the fragments of the collagen components is preferably in the range of 50 nm to 30 µm, more preferably 4 µm to 30 µm, and even more preferably 20 µm to 30 µm.

At least a portion of the extracellular matrix component may be intermolecularly or intramolecularly crosslinked. The extracellular matrix molecules forming the extracellular matrix component may be intramolecularly or intermolecularly crosslinked. When the extracellular matrix component includes a fragmented extracellular matrix component, at least a portion of the fragmented extracellular matrix component may be intermolecularly or intramolecularly crosslinked.

An extracellular matrix component that is at least partially intermolecularly or intramolecularly crosslinked can be produced, for example, by a method including crosslinking the extracellular matrix component (crosslinking step). The extracellular matrix component can include, for example, a fragmented and crosslinked extracellular matrix component. A fragmented and crosslinked extracellular matrix component can be produced, for example, by a method including fragmenting an extracellular matrix component and then crosslinking the fragmented extracellular matrix component, or by a method including crosslinking an extracellular matrix component and then fragmenting the crosslinked extracellular matrix component.

Examples of crosslinking methods include, but are not limited to, physical crosslinking by application of heat, ultraviolet light, radiation, or the like, and chemical crosslinking using a crosslinking agent, enzyme reaction, or the like. In order not to interfere with cell growth, physical crosslinking is preferred. The crosslinks (physical crosslinks and chemical crosslinks) may be crosslinks via covalent bonds.

When the extracellular matrix component includes a collagen component, the crosslinks may be formed between collagen molecules (triple helix structures) or between collagen fibrils formed by the collagen molecules. The crosslinking may be crosslinking by heat (thermal crosslinking). Thermal crosslinking can be carried out, for example, by carrying out heat treatment under reduced pressure using a vacuum pump. When a collagen component is subjected to thermal crosslinking, the extracellular matrix component may be crosslinked by forming peptide bonds (-NH-CO-) between amino groups of collagen molecules and carboxy groups of the same or other collagen molecules.

The extracellular matrix component can also be crosslinked by using a crosslinking agent. The crosslinking agent may be, for example, an agent capable of crosslinking a carboxyl group with an amino group, or one capable of crosslinking amino groups with each other. For example, aldehyde-based, carbodiimide-based, epoxide-based and imidazole-based crosslinking agents are preferred in terms of cost, safety, and ease of handling, and specific examples thereof include water-soluble carbodiimides such as glutaraldehyde, 1-ethyl-3-(3-dimethylaminopropyl)carbodiimide hydrochloride, and 1-cyclohexyl-3-(2-morpholinyl-4-ethyl)carbodiimide sulfonate.

The degree of crosslinking can be determined as appropriate depending on the type of extracellular matrix component, the means for crosslinking, and the like. The degree of crosslinking may be 1% or more, preferably 2% or more, more preferably 4% or more, even more preferably 8% or more, and still even more preferably 12% or more, and may be 30% or less, preferably 20% or less, and more preferably 15% or less. When the degree of crosslinking is within this range, the extracellular matrix molecules can be dispersed appropriately and also have good redispersibility after dry storage.

When amino groups in the extracellular matrix component are used for crosslinking, the degree of crosslinking can be quantified based on the TNBS (2,4,6-trinitrobenzenesulfonic acid) method described in Acta Biomaterialia, 2015, vol. 25, pp. 131-142, etc. The degree of crosslinking as measured by the TNBS method may be within the above range. The degree of crosslinking as measured by the TNBS method is the ratio of amino groups used for crosslinking to the total amino groups in the extracellular matrix. When the extracellular matrix component includes a collagen component, the degree of crosslinking as measured by the TNBS method is preferably within the above range.

It is also possible to calculate the degree of crosslinking by quantifying the carboxyl groups. For example, a water-insoluble extracellular matrix component may be quantified by the TBO (Toluidine Blue O) method. The degree of crosslinking as measured by the TBO method may be within the above range.

In the crosslinking step, the temperature (heating temperature) and time (heating time) for heating the extracellular matrix component can be determined as appropriate. For example, the heating temperature may be 100°C or higher and 200°C or lower, and preferably 220°C or lower. Specifically, the heating temperature may be, for example, 100°C, 110°C, 120°C, 130°C, 140°C, 150°C, 160°C, 170°C, 180°C, 190°C, 200°C, or 220°C. The heating time (the time for which the heating temperature is maintained) can be appropriately set depending on the heating temperature. For example, when heating is performed at 100°C to 200°C, the heating time may be 6 hours to 72 hours, more preferably 24 hours to 48 hours. In the crosslinking step, heating may be performed in the absence of a solvent and/or under reduced pressure.

The content of extracellular matrix component in the three-dimensional hepatic tissue relative to the dry weight of the three-dimensional hepatic tissue may be 0.01% by mass or more, preferably 0.05% by mass or more, more preferably 0.1% by mass or more, even more preferably 0.5% by mass or more, still even more preferably 1% by mass or more, still even more preferably 2% by mass or more, still even more preferably 3% by mass or more, still even more preferably 4% by mass or more, still even more preferably 5% by mass or more, still even more preferably 6% by mass or more, still even more preferably 7% by mass or more, still even more preferably 8% by mass or more, still even more preferably 9% by mass or more, still even more preferably 10% by mass or more, still even more preferably 15% by mass or more, still even more preferably 20% by mass or more, still even more preferably 25% by mass or more, and still even more preferably 30% by mass or more, and may be 90% by mass or less, preferably 80% by mass or less, more preferably 70% by mass or less, even more preferably 60% by mass or less, still even more preferably 50% by mass or less, still even more preferably 30% by mass or less, still even more preferably 20% by mass or less, and still even more preferably 15% by mass or less. The content of the extracellular matrix component in the three-dimensional hepatic tissue may be 0.01 to 90% by mass, preferably 10 to 90% by mass, more preferably 10 to 80% by mass, even more preferably 10 to 70% by mass, still even more preferably 10 to 60% by mass, still even more preferably 1 to 50% by mass, still even more preferably 10 to 50% by mass, still even more preferably 10 to 30% by mass, and still even more preferably 20 to 30% by mass, relative to the dry weight of the three-dimensional hepatic tissue.

The proportion of a collagen component in the three-dimensional hepatic tissue may be defined by their area ratio or volume ratio. "Defined by their area ratio or volume ratio" means, for example, first making the collagen component in the three-dimensional hepatic tissue distinguishable from other tissue components using a known staining technique (e.g., immunostaining with an anti-collagen antibody or Masson's trichrome staining), and then calculating the proportion of the area in which the collagen component is present in the entire area of the three-dimensional hepatic tissue by direct observation or using any of various microscopes, image analysis software, and the like. When its proportion is defined based on the area ratio, the cross section or surface in the three-dimensional hepatic tissue used to define the area ratio is not particularly limited. For example, when the three-dimensional hepatic tissue is a sphere or the like, the area ratio may be defined by a cross section passing through approximately the center thereof.

For example, when proportion of a collagen component in the three-dimensional hepatic tissue is defined by their area ratio, it is typically 0.01 to 99% relative to the total area of the three-dimensional hepatic tissue, and may be 1 to 99%, preferably 5 to 90%, more preferably 7 to 90%, even more preferably 20 to 90%, still even more preferably 30 to 90%, and still even more preferably 50 to 90%. The proportion of the area of the collagen component can be calculated, for example, by staining the obtained three-dimensional hepatic tissue with Masson's trichrome and calculating the ratio of the area of the collagen component stained blue to the total area of a cross-section passing through approximately the center of the three-dimensional hepatic tissue.

In one embodiment, the three-dimensional hepatic tissue may include a polyelectrolyte. A polyelectrolyte is a polymer compound that has properties of an electrolyte. Examples of polyelectrolytes include, but are not limited to, glycosaminoglycans such as heparin, chondroitin sulfate (e.g., chondroitin 4-sulfate and chondroitin 6-sulfate), heparan sulfate, dermatan sulfate, keratan sulfate, and hyaluronic acid; dextran sulfate, rhamnan sulfate, fucoidan, carrageenan, polystyrene sulfonic acid, polyacrylamide-2-methylpropanesulfonic acid, and polyacrylic acid, and derivatives thereof. These polyelectrolytes may be used singly or in combination of two or more.

The polyelectrolyte is preferably a glycosaminoglycan, more preferably contains at least one selected from the group consisting of heparin, dextran sulfate, chondroitin sulfate, and dermatan sulfate, and further preferably contains heparin. When the three-dimensional hepatic tissue contains a polyelectrolyte, excessive aggregation of the extracellular matrix component can be more effectively suppressed, and as a result, it becomes easier to obtain a three-dimensional hepatic tissue that is highly responsive to hepatotoxic substances. This effect is enhanced when the three-dimensional hepatocyte contains heparin.

The ratio of mass C2 of the polyelectrolyte to mass C1 of the extracellular matrix component (C2/C1) may be 1/100 to 100/1, preferably 1/10 to 10/1, more preferably 1/5 to 5/1, even more preferably 1/2 to 2/1, and still even more preferably may be 1/1.5 to 1.5/1.

The three-dimensional hepatic tissue according to this embodiment preferably includes bile canaliculi. By "includes bile canaliculi", it is meant that bile canaliculi are spaces between cells including hepatocytes, in particular, spaces between hepatocytes in contact with each other. It is known that bile acids taken up by hepatocytes are excreted into the bile canaliculi in vivo. Whether the three-dimensional hepatic tissue includes bile canaliculi can be confirmed, for example, by expression of multidrug resistance-associated protein 2 (MRP2) or bile salt export pump (BSEP), which are bile canalicular transporters. The expression of MRP2 can be confirmed by the method described in the examples below. Whether the three-dimensional hepatic tissue includes bile canaliculi can also be confirmed, for example, by bringing an optically detectable substance into contact with the three-dimensional hepatic tissue and detecting the substance retained in the bile canaliculi. The retention of an optically detectable substance in the bile canaliculi can be confirmed, for example, by a method described in the examples below. Although the formation of the bile canaliculus structure can be detected by evaluating the expression of transporters, to determine whether the structure has functions such as excretion of bile acids, it is preferable to evaluate the retention of an optically detectable substance in the bile canaliculus.

When the three-dimensional hepatic tissue of this embodiment includes bile canaliculi, and, for example, an optically detectable substance is brought into contact with the three-dimensional hepatic tissue and a signal of the substance retained in the bile canaliculi is detected, the observed signal may be a linear or mesh-like signal. In the three-dimensional hepatic tissue of this embodiment, the occurrence rate of linear or mesh-like signals having a length of 100 µm or more may be 5% or more, preferably 10% or more, and more preferably 15% or more. The three-dimensional hepatic tissue of this embodiment may have a total bile canaliculus length per tissue area of 25,000 µm/mm² or more, preferably 26,000 µm/mm² or more, more preferably 27,000 µm/mm² or more, even more preferably 28,000 µm/mm² or more, still even more preferably 29,000 µm/mm² or more, still even more preferably 30,000 µm/mm² or more, still even more preferably 31,000 µm/mm² or more, still even more preferably 32,000 µm/mm² or more, still even more preferably 33,000 µm/mm² or more, still even more preferably 34,000 µm/mm² or more, and still even more preferably 35,000 µm/mm² or more.

The three-dimensional hepatic tissue of this embodiment may have an amount of albumin secretion on day 28 of culture of 5 µg/10⁶ cells/day or more, preferably 10 µg/10⁶ cells/day or more, more preferably 15 µg/10⁶ cells/day or more, and even more preferably 20 µg/10⁶ cells/day or more. The amount of albumin secreted by the three-dimensional hepatic tissue can be evaluated, for example, by measuring it using a method described in the examples below. Specifically, the evaluation can be performed by measuring albumin in the culture supernatant using ELISA.

In the three-dimensional hepatic tissue of this embodiment, blood vessels may be formed between at least some of the cells. "Blood vessels are formed between cells" means that the tubular structures formed by vascular endothelial cells extend between the cells.

The three-dimensional hepatic tissue of this embodiment may have a hepatic sinusoidal network between the cells. "Have a hepatic sinusoidal network" means that the tubular structures formed by vascular endothelial cells are branched so as to form a mesh-like structure surrounding the cells. The presence of intercellular vascular structures and hepatic sinusoidal network can be confirmed by immunohistochemical staining. For example, the three-dimensional hepatic tissue may have a hepatic sinusoidal network to the extent that it can be observed to have a plurality of branching points and form a network structure when observed from above with a microscope.

The thickness of the three-dimensional hepatic tissue may be 10 µm or more, preferably 30 µm or more, more preferably 50 µm or more, even more preferably 100 µm or more, still even more preferably 300 µm or more, and still even more preferably 1,000 µm or more. Such three-dimensional hepatic tissue has a structure similar to that of biological tissue and is suitable as a substitute for laboratory animals and as a transplant material. The upper limit of the thickness of the three-dimensional hepatic tissue is not particularly limited, but may be, for example, 10 mm or less, preferably 3 mm or less, more preferably 2 mm or less, even more preferably 1.5 mm or less, still even more preferably 1 mm or less, still even more preferably 300 µm or less, still even more preferably 200 µm or less, still even more preferably 150 µm or less, and still even more preferably 100 µm or less. By reducing the maximum thickness of the three-dimensional hepatic tissue while maintaining the three-dimensional structure, it is possible to form a three-dimensional hepatic tissue whose interior can be easily observed and that can be sufficiently supplied with nutrients.

Here, when the three-dimensional hepatic tissue is a rectangular prism, the "thickness of the three-dimensional hepatic tissue" refers to the distance between both ends thereof in the direction perpendicular to its main surface. When the main surface is uneven, the thickness means the distance at the thinnest part of the main surface.

When the three-dimensional hepatic tissue is spherical or approximately spherical, the thickness of the three-dimensional hepatic tissue means the diameter thereof. When the three-dimensional hepatic tissue is ellipsoidal or approximately ellipsoidal, the thickness of the three-dimensional hepatic tissue means the minor axis thereof. When the three-dimensional hepatic tissue is approximately spherical or approximately ellipsoidal and has an uneven surface, the thickness of the three-dimensional hepatic tissue means the shortest distance between the two points where a line passing through the center of gravity thereof intersects with the surface.

The three-dimensional hepatic tissue according to the present embodiment is produced in a cell culture vessel. The cell culture vessel is not particularly limited, as long as it enables the production of three-dimensional hepatic tissue and enables the culture of the produced three-dimensional hepatic tissue. Specifically, examples of the cell culture vessel include dishes, cell culture inserts (e.g., Transwell (registered trademark) inserts, Netwell (registered trademark) inserts, Falcon (registered trademark) cell culture inserts, and Millicell (registered trademark) cell culture inserts), tubes, flasks, bottles, plates, and the like. In the production of the three-dimensional hepatic tissue, dishes or various cell culture inserts are preferable, because they can more appropriately allow evaluation using the three-dimensional hepatic tissue.

### [Method of manufacturing three-dimensional hepatic tissue]

The method of manufacturing three-dimensional hepatic tissue according to this embodiment includes causing a composition containing cells and a hydrogel-forming substance to gel so that the cells are embedded in the hydrogel (gelation step), and culturing the cells embedded in the hydrogel (culturing step). The cells include at least hepatocytes and do not include hepatic stellate cells. The cells may include cells other than hepatocytes. The other cells can be those described above.

The hydrogel-forming substance is a substance that forms a hydrogel in response to some external stimulus such as a chemical stimulus or a physical stimulus, and that is not in a hydrogel state. Examples of hydrogels are provided above, and examples of hydrogel-forming substances include fibrinogen and thrombin, collagen, gelatin, hyaluronic acid, alginic acid, and pectin. The hydrogel-forming substance preferably contains fibrinogen and thrombin. Fibrin is a component that is generated when thrombin acts on fibrinogen to release A and B chains from the N-termini of Aα and Bβ chains. Fibrin is formed by contacting fibrinogen with thrombin.

The concentration of the hydrogel-forming substance in the composition containing the cells and the hydrogel-forming substance may be 0.1 mg/mL or more, preferably 0.2 mg/mL or more, more preferably 0.3 mg/mL or more, even more preferably 0.4 mg/mL or more, still even more preferably 0.5 mg/mL or more, still even more preferably 0.6 mg/mL or more, still even more preferably 0.7 mg/mL or more, still even more preferably 0.8 mg/mL or more, still even more preferably 0.9 mg/mL or more, and still even more preferably 1.0 mg/mL or more, relative to the total amount of the aqueous medium. The concentration of the hydrogel-forming substance when contacted with the cells in the aqueous medium may be 10.0 mg/mL or less, preferably 8.0 mg/mL or less, more preferably 6.0 mg/mL or less, even more preferably 5.0 mg/mL or less, still even more preferably 3.0 mg/mL or less, still even more preferably 1.0 mg/mL or less, still even more preferably 0.8 mg/mL or less, and still even more preferably 0.6 mg/mL or less, relative to the total amount of the aqueous medium.

For example, when the hydrogel-forming substance includes two or more substances, such as a combination of fibrinogen and thrombin, a composition containing cells and the hydrogel-forming substances may be obtained, for example, by mixing a first composition containing the cells and a first hydrogel-forming substance (e.g., thrombin) with a second composition containing a second hydrogel-forming substance (e.g., fibrinogen). The second composition may contain the cells, or both the first and second compositions may contain cells.

The gelation of a composition containing cells and a hydrogel-forming substance may be carried out in an aqueous medium. The term "aqueous medium" refers to a liquid containing water as an essential component. The aqueous medium may be, for example, an aqueous medium containing a cationic substance. The aqueous medium containing a cationic substance may be, for example, a cationic buffer solution such as a tris-hydrochloric acid buffer, tris-maleic acid buffer, Bis-Tris buffer, or 4-(2-hydroxyethyl)-1-piperazineethanesulfonic acid (HEPES), or may be a medium containing water and a cationic compound such as ethanolamine, diethanolamine, triethanolamine, polyvinylamine, polyallylamine, polylysine, polyhistidine, or polyarginine as the cationic substance. A medium can also be used as the aqueous medium. Examples of the medium include liquid media such as Dulbecco's Modified Eagle's Medium (DMEM) and hepatocyte culture media (HCM). The liquid medium may be a mixed medium in which two types of media are mixed.

The concentration and pH of the cationic substance in an aqueous medium containing the cationic substance (e.g., tris in a tris-hydrochloric acid buffer solution) are not specifically limited as long as they do not adversely affect the growth of the cells and production of the three-dimensional hepatic tissue. For example, the concentration of the cationic substance relative to the total amount of the aqueous medium containing the cationic substance may be 10 to 100 mM, preferably 40 to 70 mM, and more preferably may be 50 mM. The pH of the aqueous medium (e.g., cationic buffer) may be 6.0 to 8.0, preferably 6.8 to 7.8, and more preferably 7.2 to 7.6.

Gelation of the composition containing cells and a hydrogel-forming substance may include incubation for a certain period of time.

The composition containing cells and a hydrogel-forming substance may further contain an extracellular matrix component. Examples of extracellular matrix components are provided above.

In one embodiment of the method of manufacturing three-dimensional hepatic tissue, when embedding a cell structure containing hepatocytes in a hydrogel, the cells may first be suspended once in an aqueous medium containing an extracellular matrix component and a cationic substance. After removing the aqueous medium, the cells may be resuspended in an aqueous medium containing a hydrogel-forming substance to prepare a composition containing the hydrogel-forming substance. It is expected that this step facilitates aggregation of cells inside the hydrogel, contributing to the formation of even more densely packed bile canaliculi.

The composition containing cells and a hydrogel-forming substance may further contain a polyelectrolyte.

### Examples of polyelectrolytes are provided above.

In one embodiment of the method of manufacturing three-dimensional hepatic tissue, when embedding a cell structure containing hepatocytes in a hydrogel, the cells may first be suspended once in an aqueous medium containing an extracellular matrix component, a polyelectrolyte, and a cationic substance. After removing the aqueous medium, the cells may be resuspended in an aqueous medium containing a hydrogel-forming substance to prepare a composition containing the hydrogel-forming substance. It is expected that this step facilitates aggregation of cells inside the hydrogel, contributing to the formation of even more densely packed bile canaliculi. In addition, when the three-dimensional hepatic tissue contains a polyelectrolyte, excessive aggregation of the extracellular matrix component can be more effectively suppressed, and as a result, it becomes easier to obtain a three-dimensional hepatic tissue that is highly responsive to hepatotoxic substances.

The cells in the gelation step may be aggregated cells. In other words, the gelation step may be a step of causing a composition containing aggregated cells and a hydrogel-forming substance to gel so that the aggregated cells are embedded in the hydrogel. By performing a gelation process using aggregated cells, it becomes easy to produce three-dimensional hepatic tissue in which the exterior of the cell structure is covered with hydrogel and no hydrogel is present in the intercellular spaces inside the cell structure. The cells can be aggregated by centrifugation, natural sedimentation, or the like. Alternatively, even if the cells are not completely aggregated when embedded in the hydrogel-forming substance, aggregation of the cells may progress inside simultaneously with the gelation. In a case where cell aggregation progresses inside the hydrogel-forming substance simultaneously with the gelation, the cells are more likely to adhere to the support while they adhere to each other, facilitating the formation of an approximately dome-shaped three-dimensional hepatic tissue.

If necessary, after the gelation step, a freezing treatment may be carried out by adding a cryoprotectant or the like to the vessel in which the hydrogel embedded with cells has been formed. Even if the gel is frozen immediately after the gelation step and then thawed, the gel would not be damaged, and the culture and formation of three-dimensional hepatic tissue can be continued after thawing without causing the embedded cells to be scattered. The procedures for the freezing treatment are not particularly limited, but for example, it can be carried out by placing the plate in an environment at a temperature of -80°C, -150°C, or lower. The freezing period is not particularly limited, but for example, the frozen storage can be carried out for one day, one week, two weeks, one month, two months, three months, or half a year. The procedures for thawing are not particularly limited, but for example, thawing may be performed by removing the plate from the frozen storage environment, adding a medium that has been warmed to 37°C in advance to the wells, and leaving the plate in an incubator for 10 minutes or more, 20 minutes or more, 30 minutes or more, or 60 minutes or more. If a cryoprotectant was added in the freezing step, if there is a concern that the reagent may be toxic to the cells, it is preferable to change the medium promptly after the thawing.

In the culture step, the cells are cultured embedded in the hydrogel. Culturing can be carried out, for example, by adding a medium to the hydrogel in which the cells are embedded.

An example of the medium used in the culture step is a medium for hepatocytes (e.g., an HCM (manufactured by Lonza)). The medium may be a medium supplemented with serum or a serum-free medium. In particular, a medium that does not contain fetal bovine serum (FBS) may be used. The medium may be a medium supplemented with growth factors. The medium may be a mixed medium in which two types of media are mixed.

The culture step may be carried out in the presence of a pro-angiogenic factor when the cells include vascular endothelial cells. A medium containing a pro angiogenic factor may be used as the medium for culturing the cells. Examples of pro-angiogenic factors include vascular endothelial growth factor (VEGF) and fibroblast growth factor (FGF).

The culture temperature in the culture step may be, for example, 20°C to 40°C, and preferably 30°C to 37°C. The pH of the medium may be 6 to 8, and preferably 7.2 to 7.4. The culture time may be 1 day to 4 weeks, preferably 1 day to 2 weeks, and more preferably 1 week to 2 weeks.

The culture vessel (support) is not particularly limited and may be, for example, a dish, a well insert, a low-adhesion plate, or a plate with a U- or V-shaped bottom. The cells may be cultured attached the support, or may be cultured without being attached to the support, or may be detached from the support during the culture. When the cells are cultured without being attached to the support, or when they are detached from the support during the culture, it is preferable to use a plate having a bottom shape such as a U- or V-shape that inhibits adhesion of the cells to the support, or a low-adhesion plate.

The cell density in the medium in the culture step can be appropriately determined depending on the desired shape and thickness of the three-dimensional hepatic tissue, the size of the culture vessel, and the like. For example, the cell density in the medium in the culture step may be 1 to 10⁸ cells/mL, and preferably 10³ to 10⁷ cells/mL. Further, the cell density in the medium in the culture step may be the same as that in the aqueous medium in the contacting step.

### [Applications of the three-dimensional hepatic tissue]

The three-dimensional hepatic tissue of this embodiment can be used as a substitute for experimental animals, transplant material, and the like. Specific examples of its application include tissue reproduction, pathological in vitro models, pharmaceutical screening (drug evaluation), and assay screening of cosmetic products.

### Examples

In the following, the present invention will be described in more detail by way of examples. However, the present invention is not limited to these.

The present invention will be described more specifically and in more detail by way of the Examples. However, they should not limit the scope of the present invention. In the following examples, type I collagen (manufactured by Nippi) was used as the collagen unless otherwise specified.

### [Test Example 1: Production of three-dimensional hepatic tissue and evaluation of metabolic enzyme expression]

### <Condition 1: Three-dimensional hepatic tissue consisting only of hepatocytes>

5.85 × 10⁵ human hepatocytes (PXB cells (registered trademark)) were suspended in an equal volume mixture of 50 µL of 1.0 mg/mL heparin/200 mM Tris-HCl buffer (pH 7.4) and 50 µL of 0.6 mg/mL collagen/5 mM acetic acid solution (pH 3.7) (heparin-collagen solution). The resulting mixture was centrifuged at room temperature at 400 × g for 2 minutes, the supernatant was removed, and the cells were resuspended in an HCM (product number "CC-3198", manufactured by Lonza) containing 10 U/mL thrombin (product number "R4648", manufactured by Sigma-Aldrich) and 1 v/v% endothelial cell growth supplement (ECGS) (product number "1052", manufactured by Sciencell) to a cell density of 1.95 × 10⁴ cells/2 µL to obtain a cell suspension.

2 µL of an HCM containing 5 mg/mL fibrinogen and 1 v/v% ECGS was added to a 48-well microplate (product number "3830-048", manufactured by AGC Techno Glass) to form a droplet. The cell suspension was added to the interior of each droplet, and the 48-well microplate was then left to stand in an incubator for 40 minutes to form a fibrin gel in which the human hepatocytes were embedded. 0.5 mL of HCM containing 1 v/v% ECGS was added to each well in which the fibrin gel had been formed, and the cells were cultured under the conditions of 37°C and 5% CO₂. After the start of the culture, the medium was changed every two days.

RNA was extracted from the three-dimensional hepatic tissue on days 1, 4, and 8 of culture. RNA was extracted using Direct-zol RNA Kit (product number "R2061", manufactured by Zymo Research). The extracted RNA was subjected to a reverse transcription reaction to synthesize complementary DNA (cDNA). The synthesis of cDNA was carried out using SuperScript (registered trademark) VILO (registered trademark) cDNA Synthesis Kit (product number "11754050", manufactured by Thermo Fisher). The synthesized cDNA was subjected to quantitative PCR analysis to analyze the expression levels of CYP3A4, CYP2C9, and GAPDH. The quantitative PCR analysis was carried out using Taqman (registered trademark) Fast Advanced Master Mix (product number "4444556", manufactured by Thermo Fisher) and Taqman (registered trademark) Gene Expression assay (product number "4331182", manufactured by Thermo Fisher). As primers for quantitative PCR, oligonucleotides with assay ID: Hs00604506_m1 were used for CYP3A4, with assay ID: Hs00426397_m1 for CYP2C9, and with assay ID: Hs99999905_m1 for GAPDH. A StepOnePlus real-time PCR system (Thermo Fisher) was used as the analytical device, and the analysis was performed according to the thermal profile recommended by the manufacturer.

### <Condition 2: Three-dimensional hepatic tissue consisting of hepatocytes and sinusoidal endothelial cells>

A cell mixture of 5.85 × 10⁵ human hepatocytes (PXB cells (registered trademark)) and 2.25 × 10⁵ sinusoidal endothelial cells (SEC) was suspended in a heparin-collagen solution as in Condition 1. The resulting mixture was centrifuged at 400 × g for 2 minutes at room temperature, the supernatant was removed, and the cells were resuspended in an HCM containing 10 U/mL thrombin and 1 v/v% ECGS to a cell density of 2.7 × 10⁴ cells/2 µL to obtain a cell suspension. Then, three-dimensional hepatic tissue was produced, RNA was extracted, and quantitative PCR analysis was performed in the same manner as in Condition 1.

### <Condition 3: Three-dimensional hepatic tissue consisting of hepatocytes and hepatic stellate cells>

A cell mixture of 5.85 × 10⁵ human hepatocytes (PXB cells (registered trademark)) and 9.0 × 10⁴ hepatic stellate cells (LX2) was suspended in a heparin-collagen solution as in Condition 1. The resulting mixture was centrifuged at 400 × g for 2 minutes at room temperature, the supernatant was removed, and the cells were resuspended in an HCM containing 10 U/mL thrombin and 1 v/v% ECGS to a cell density of 2.25 × 10⁴ cells/2 µL to obtain a cell suspension. Then, three-dimensional hepatic tissue was produced, RNA was extracted, and quantitative PCR analysis was performed in the same manner as in Condition 1.

### <Condition 4: Three-dimensional hepatic tissue consisting of hepatocytes, sinusoidal endothelial cells, and hepatic stellate cells>

A cell mixture of 5.85 × 10⁵ human hepatocytes (PXB cells (registered trademark)), 2.25 × 10⁵ sinusoidal endothelial cells (SEC), and 9.0 × 10⁴ hepatic stellate cells (LX2) was suspended in a heparin-collagen solution as in Condition 1. The resulting mixture was centrifuged at 400 × g for 2 minutes at room temperature, the supernatant was removed, and the cells were resuspended in an HCM containing 10 U/mL thrombin and 1 v/v% ECGS to a cell density of 3.0 × 10⁴ cells/2 µL to obtain a cell suspension. Then, three-dimensional hepatic tissue was produced, RNA was extracted, and quantitative PCR analysis was performed in the same manner as in Condition 1.

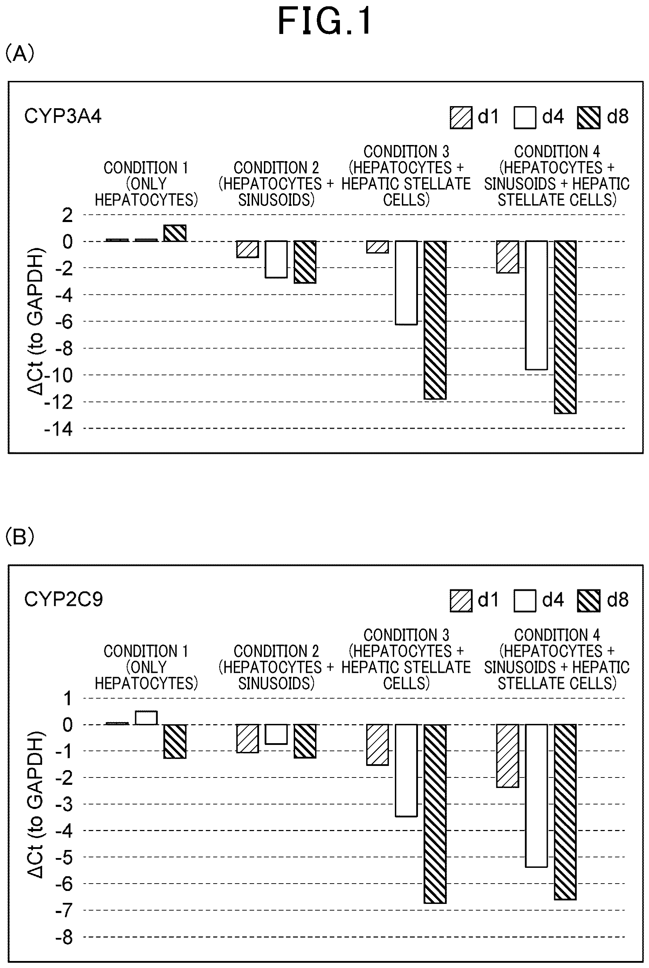

Fig. 1 is a graph showing the results of analyzing the expression levels of the CYP3A4 gene (Fig. 1(A)) and CYP2C9 gene (Fig. 1(B)). The vertical axis (ΔCt) in Fig. 1 indicates the relative number of amplification cycles (Ct value) of CYP3A4 or CYP2C9 to the Ct value of GAPDH. Since the Ct value indicates the number of PCR cycles required for the target gene product in a sample to be amplified to a detectable level, when the Ct values differ by 1, it means the expression levels differ by a factor of 2. As shown in Fig. 1, under Conditions 1 and 2, the expression levels of CYP3A4 and CYP2C9 did not change significantly from the expression levels from the initial stage of culture to day 8, indicating that liver function was maintained. On the other hand, under Conditions 3 and 4 where hepatic stellate cells were contained in the three-dimensional hepatic tissue, expression decreased rapidly, indicating that it was difficult to maintain liver function. Specifically, for CYP3A4, there was a 2¹⁰-fold or greater difference in expression level between days 1 and 8 of culture, and also for CYP2C9, there was a 2⁴- to 2⁵-fold or greater difference in expression level therebetween.

### [Test Example 2: Production of three-dimensional hepatic tissue and mRNA sequencing]

### <Condition 1: Three-dimensional hepatic tissue consisting only of hepatocytes>

After producing three-dimensional hepatic tissue in the same manner as in Condition 1 of Test Example 1, RNA was extracted on days 1, 4, and 8 of culture. We commissioned the Research Institute for Microbial Diseases, Osaka University, to perform mRNA sequencing on the extracted RNA. The read count data obtained by the sequencing was combined with the data from Conditions 2, 3, and 4 described below to normalize expression levels, and then principal component analysis, extraction of differentially expressed genes (DEGs) between different conditions, and enrichment analysis of the DEGs were performed.

### <Condition 2: Sandwich-cultured tissue consisting only of hepatocytes>

1.0 × 10⁶ human hepatocytes (PXB cells (registered trademark)) were suspended in a PXB cells culture medium (product number "PPC-M200", manufactured by Phoenix Bio) to a cell density of 2.0 × 10⁵ cells/500 µL to obtain a cell suspension. 500 µL of the cell suspension was seeded onto a Biocoat (registered trademark) collagen I 48-well microplate (product number "3545054", manufactured by Corning). Four hours after seeding, the medium was replaced with an HCM containing 1 v/v% ECGS and in which Matrigel (product number "356237", manufactured by Corning) is diluted 25-fold. After that, the medium was replaced with the same Matrigel-containing medium every two days. The produced sandwich-cultured tissue was subjected to RNA extraction, mRNA sequencing, and various gene expression analyses in the same manner as in Condition 1 of Test Example 2.

### <Condition 3: Spheroids consisting only of hepatocytes>

5.0 × 10⁵ human hepatocytes (PXB cells (registered trademark)) were suspended in an HCM containing 1 v/v% ECGS to a cell density of 1.0 × 10⁵ cells/200 µL to obtain a cell suspension. 200 µL of the cell suspension was seeded onto an EZSPHERE (registered trademark) 96-well plate (product number "4860-900", manufactured by AGC Techno Glass). After that, the medium was changed every two days. A plurality of spheroids formed in one well were collected and subjected to RNA extraction, mRNA sequencing, and various gene expression analyses in the same manner as in Condition 1 of Test Example 2.

### <Condition 4: RNA extracted from human liver>

Commercially available RNA samples extracted from human liver (the three types specified below) were subjected to mRNA sequencing and various gene expression analyses in the same manner as in Condition 1 of Test Example 2.
- Product number "636531", manufactured by Clonteck
- Product number "AM7960", manufactured by Thermo Fisher
- Product number "540017", manufactured by Agilent

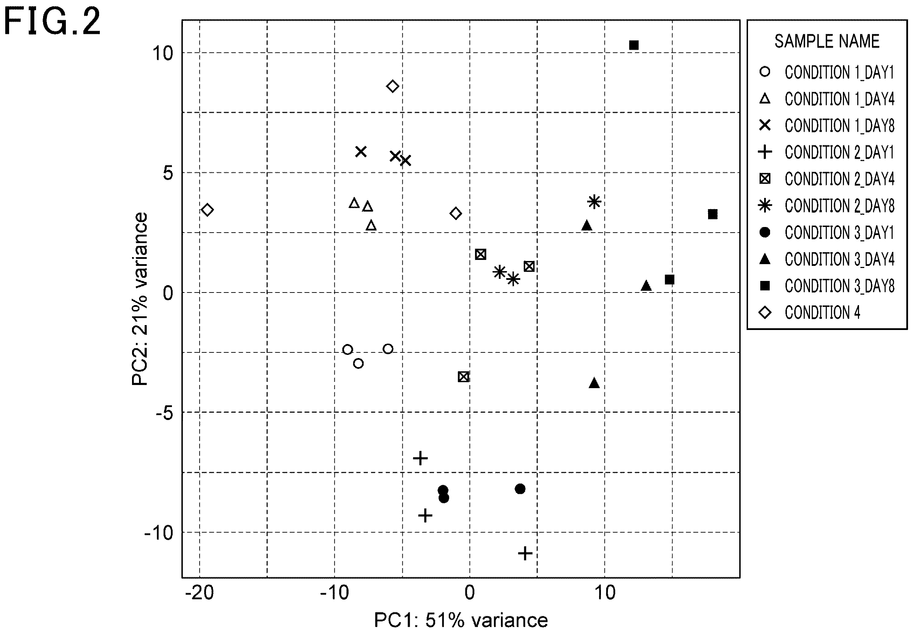

Fig. 2 is a diagram showing the results of the principal component analysis. The principal component analysis shown in Fig. 2 was performed on 61 genes shown in Table 1 below, which are considered to be particularly highly expressed in hepatocytes in the actual liver and to be related to the metabolic functions of the liver.

**[Table 1]**

| | | | | | | |
|---|---|---|---|---|---|---|
| GSTA5 | ADH1C | CYP39A1 | CYP2C8 | ALDH1A1 | GSTZ1 | DHRS12 |
| CYP2A6 | ADH1A | SULT1A2 | PON1 | ALDH5A1 | ABCC6 | |
| CYP2A7 | ADH1B | XDH | FMO3 | ALDH1B1 | GPX4 | |
| SULT1E1 | EPHX1 | MAT1A | FMO5 | CYP4F2 | CAT | |
| CYP3A4 | CYP4A11 | SLCO1A2 | ALDH2 | CYP27A1 | NR1I3 | |
| CYP2A13 | CYP2C19 | SLCO1B3 | CYP2C9 | HAGH | NR1I2 | |
| UGT2B10 | SLC22A1 | UGT2B15 | DHRS1 | EPHX2 | UGT1A9 | |
| CYP1A2 | SLCO4C1 | ADH6 | UGT2B7 | NAT1 | CHST13 | |
| CYP3A43 | SLC22A9 | SLC22A7 | CYP4F3 | FMO4 | DHRS4 | |
| ADH4 | GSTA1 | SULT1A1 | ABCG2 | SLC15A1 | GSTK1 | |

As shown in Fig. 2, the gene expression pattern of the three-dimensional hepatic tissue under Condition 1, which is an example of the present invention, had a tendency to become closer to that of the human liver tissue under Condition 4 from day 1 to day 8 of culture. On the other hand, the expression patterns of the sandwich-cultured tissue under Condition 2 and the spheroids under Condition 3 did not become closer to that of the human liver tissue under Condition 4 even when their culture was continued.

Next, DEGs were extracted between the three-dimensional hepatic tissue under Condition 1 and the sandwich-cultured tissue under Condition 2, the spheroids under Condition 3, or the human liver tissue under Condition 4 on day 8 of culture, and enrichment analysis was performed on the DEGs. DEG extraction was performed on 1,156 genes that are considered to be relatively highly expressed in hepatocytes in the actual liver. Further analysis was performed using the DESeq2 package, and genes with FDR < 0.05 and fold change > 2 were extracted as DEGs. The results are shown in Tables 2 to 5. The enrichment analysis was performed with reference to the KEGG database.

**[Table 2]**

| | | | |
|---|---|---|---|
| No. of DEGs with significantly higher expression levels under condition 1 | | 385 | |
| No. of DEGs with significantly higher expression levels under condition 2 | | 44 | |

| | adj. Pval | No. of DEGs involved | Enhanced pathways |
|---|---|---|---|
| Biological pathways related to DEGs with high expression levels under condition 1 | 8.70E-75 | 151 | Metabolic pathways |
| | 1.90E-25 | 25 | Retinol metabolism |
| | 2.10E-23 | 21 | Valine, leucine and isoleucine degradation |
| | 1.60E-22 | 23 | **Drug metabolism** |
| | 2.70E-21 | 22 | Chemical carcinogenesis |
| | 1.30E-19 | 28 | Biosynthesis of cofactors |
| | 2.60E-18 | 17 | Fatty acid degradation |
| | 8.60E-18 | 20 | Metabolism of xenobiotics by cytochrome P450 |
| | 4.10E-16 | 22 | **Carbon metabolism** |
| | 1.20E-14 | 16 | **Fatty acid metabolism** |
| | 3.20E-14 | 18 | Complement and coagulation cascades |
| | 3.30E-14 | 16 | Steroid hormone biosynthesis |
| | 1.50E-13 | 17 | **Drug metabolism** |
| | 2.20E-13 | 14 | **Pyruvate metabolism** |
| | 6.20E-13 | 12 | **Propanoate metabolism** |
| Biological pathways related to DEGs with high expression levels under condition 2 | None | | |

According to the results in Table 2, which shows a comparison between the three-dimensional hepatic tissue under Condition 1 and the sandwich-cultured tissue under Condition 2, the number of DEGs whose expression was significantly higher in the three-dimensional hepatic tissue under Condition 1 was 385, whereas the number of DEGs whose expression was significantly higher in the sandwich-cultured tissue under Condition 2 was 44. This means that the three-dimensional hepatic tissue under Condition 1 showed increased expression of more genes compared to the sandwich-cultured tissue under Condition 2.

Further, the results of the enrichment analysis shown in Table 2 indicate that many of the biological pathways associated with DEGs whose expression was increased in the three-dimensional hepatic tissue under Condition 1 were related to metabolism. On the other hand, no biological pathways associated the DEGs whose expression was increased in the sandwich-cultured tissues under Condition 2 were identified based on the KEGG database. These results demonstrate that, in the three-dimensional hepatic tissue under Condition 1, various biological pathways, mainly metabolic pathways, were more activated than in the sandwich-cultured tissue under Condition 2.

**[Table 3]**

| | |
|---|---|
| No. of DEGs with significantly higher expression levels under condition 1 | 149 |
| No. of DEGs with significantly higher expression levels under condition 3 | 0 |

| | adj. Pval | No. of DEGs involved | Enhanced pathways |
|---|---|---|---|
| Biological pathways related to DEGs with high expression levels under condition 1 | 9.7E-20 | 49 | **Metabolic pathways** |
| | 5E-13 | 12 | **Retinol metabolism** |
| | 5E-13 | 12 | Chemical carcinogenesis |
| | 1.5E-11 | 11 | **Drug metabolism** |
| | 1.9E-08 | 9 | Metabolism of xenobiotics by cytochrome P450 |
| | 2.9E-08 | 9 | **Drug metabolism** |
| | 4.3E-08 | 9 | Complement and coagulation cascades |
| | 5E-08 | 8 | Steroid hormone biosynthesis |
| | 5E-08 | 11 | Biosynthesis of cofactors |
| | 2.7E-07 | 6 | **Ascorbate and aldarate metabolism** |
| | 5.4E-07 | 6 | Pentose and glucuronate interconversions |
| | 2.1E-06 | 6 | **Porphyrin and chlorophyll metabolism** |
| | 0.000005 | 6 | **Cholesterol metabolism** |
| | 0.00001 | 7 | Bile secretion |
| | 0.000013 | 3 | **Caffeine metabolism** |
| Biological pathways related to DEGs with high expression levels under condition 3 | None | | |

According to the results in Table 3, which shows a comparison between the three-dimensional hepatic tissue under Condition 1 and the spheroids under Condition 3, the number of DEGs whose expression was significantly higher in the three-dimensional hepatic tissue under Condition 1 was 149, whereas the number of DEGs whose expression was significantly higher in the spheroids under Condition 3 was 0. This means that the three-dimensional hepatic tissue under Condition 1 showed increased expression of more genes compared to the spheroids under Condition 3.

Further, similarly to the results in Table 2, the results of the enrichment analysis indicate that many of the biological pathways associated with DEGs whose expression was increased in the three-dimensional hepatic tissue under Condition 1 were related to metabolism. On the other hand, no biological pathways associated the DEGs whose expression was increased in the spheroids under Condition 3 were identified based on the KEGG database. These results demonstrate that, in the three-dimensional hepatic tissue under Condition 1, various biological pathways, mainly metabolic pathways, were more activated than in the spheroids under Condition 3.

**[Table 4]**

| | |
|---|---|
| No. of DEGs with significantly higher expression levels under condition 1 | 201 |
| No. of DEGs with significantly higher expression levels under condition 4 | 253 |

| | adj. Pval | No. of DEGs involved | Enhanced pathways |
|---|---|---|---|
| Biological pathways related to DEGs with high expression levels under condition 1 | 2.6E-19 | 57 | **Metabolic pathways** |
| | 3.9E-11 | 12 | Chemical carcinogenesis |
| | 1.5E-08 | 10 | **Retinol metabolism** |
| | 7.6E-06 | 8 | Metabolism of xenobiotics by cytochrome P450 |
| | 0.000022 | 7 | Steroid hormone biosynthesis |
| | 0.000022 | 10 | Biosynthesis of cofactors |
| | 0.00013 | 5 | **Propanoate metabolism** |
| | 0.00015 | 5 | Pentose and glucuronate interconversions |
| | 0.00038 | 6 | **Drug metabolism** |
| | 0.00067 | 5 | Valine, leucine and isoleucine degradation |
| | 0.0007 | 7 | **Carbon metabolism** |
| | 0.00077 | 4 | Fatty acid elongation |
| | 0.00095 | 4 | **Linoleic acid metabolism** |
| | 0.001 | 4 | **Ascorbate and aldarate metabolism** |
| | 0.001 | 5 | **Fatty acid metabolism** |
| Biological pathways related to DEGs with high expression levels under condition 4 | 5.9E-15 | 17 | Complement and coagulation cascades |
| | 2.8E-13 | 55 | **Metabolic pathways** |
| | 0.000041 | 5 | Primary bile acid biosynthesis |
| | 0.0004 | 12 | Coronavirus disease |
| | 0.00078 | 9 | Alcoholic liver disease |
| | 0.0009 | 5 | **Tyrosine metabolism** |
| | 0.0009 | 8 | **Carbon metabolism** |
| | 0.0013 | 5 | Glycine, serine and threonine metabolism |
| | 0.0013 | 7 | Staphylococcus aureus infection |
| | 0.0014 | 5 | Fatty acid degradation |
| | 0.0014 | 6 | **Retinol metabolism** |
| | 0.0019 | 5 | **Pyruvate metabolism** |
| | 0.0061 | 5 | Steroid hormone biosynthesis |
| | 0.0072 | 4 | Alanine, aspartate and glutamate metabolism |
| | 0.0075 | 7 | Systemic lupus erythematosus |

According to the results in Table 4, which shows a comparison between the three-dimensional hepatic tissue under Condition 1 and the human liver tissue under Condition 4, the number of DEGs whose expression was significantly higher in the three-dimensional hepatic tissue under Condition 1 was 201, whereas the number of DEGs whose expression was significantly higher in the human liver tissue under Condition 4 was 253. A certain number of DEGs were observed in both the three-dimensional hepatic tissue under Condition 1 and the human liver tissue under Condition 4.

Further, the results of the enrichment analysis indicate that enhancement of biological pathways, including those related to metabolism, was observed to the same degree in the three-dimensional hepatic tissue under Condition 1 and the human liver tissue under Condition 4. From these results, it can be considered that various biological pathways including metabolic pathways are approximately equally active in the three-dimensional hepatic tissue under Condition 1 and the human liver tissue under Condition 4. Since Condition 4 is human liver tissue, it was found that the three-dimensional hepatic tissue under Condition 1 has properties that are very similar to those of liver tissue in an actual human body.

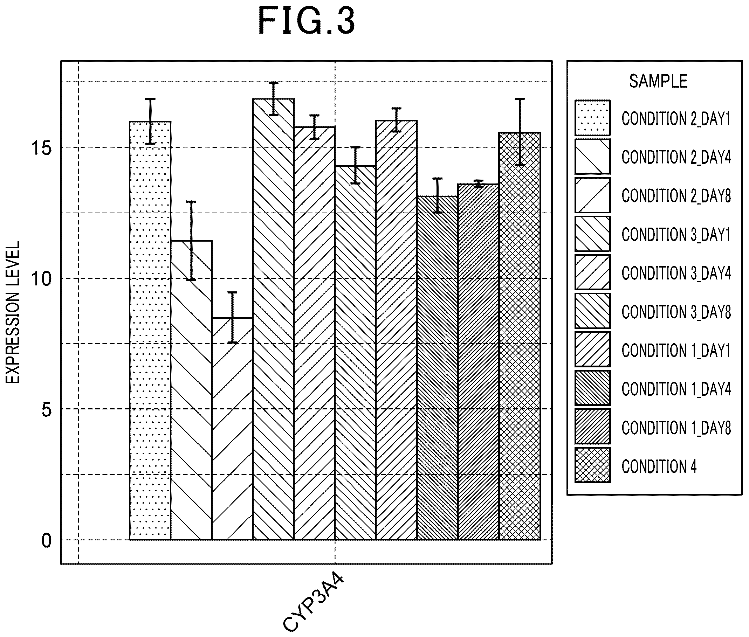

Fig. 3 is a graph showing the results of analyzing the expression level of the CYP3A4 gene, which is the most important gene for the metabolic functions of hepatocytes. In the sandwich-cultured tissue under Condition 2, the expression level of CYP3A4 decreased 100-fold or more from day 1 of culture to day 8 of culture. In contrast, in the three-dimensional hepatic tissue under Condition 1 and the spheroids under Condition 3, although there was a mild decreasing trend from day 1 to day 8, it was not a rapid, continuous decrease, and the change was no greater than 10-fold. In addition, their differences from the human liver tissue under Condition 4 were small.

### [Test Example 3: Production of three-dimensional hepatic tissue and evaluation of bile canaliculi formation]

### <Condition 1: Three-dimensional hepatic tissue consisting only of hepatocytes>

After producing three-dimensional hepatic tissue in the same manner as in Condition 1 of Test Example 2, on day 14 of culture, the medium was replaced with an HCM containing 1 v/v% ECGS, 5 µM carboxydichlorofluorescein diacetate (CDFDA, product number "22025", manufactured by AAT Bioquest), and 0.1% DMSO (product number "041-29351", manufactured by FUJIFILM Wako Pure Chemical Corporation), and the tissue was incubated in an incubator at 37°C for 20 minutes. Then, the CDFDA-containing medium was removed, and the three-dimensional hepatic tissue was washed four times with PBS. The medium was replaced again with an HCM containing 1 v/v% ECGS, and the localization of fluorescein fluorescence was evaluated using a confocal microscope. Further, on day 14 of culture, the cells were fixed with a 10% formalin neutral buffer (product number "062-01661", manufactured by FUJIFILM Wako Pure Chemical Corporation), and then immunostained with anti-MRP2 antibody (product number "ab3373", manufactured by Abcam, used at 200-fold dilution) and Alexa647-labeled anti-mouse IgG secondary antibody (product number "A-21235", manufactured by Thermo Fisher, used at 400-fold dilution) to evaluate MRP2 expression using a confocal microscope. Note that, after being taken up by hepatocytes, CDFDA is hydrolyzed into CDF, which is a fluorescent substance, and excreted into the bile canaliculi.

### <Condition 2: Sandwich-cultured tissue consisting only of hepatocytes>

Sandwich-cultured tissue was produced in the same manner as in Condition 2 of Example 2. The obtained tissue was subjected to CDFDA treatment and immunostained in the same manner as in Condition 1 of Test Example 3, and then observed using a confocal microscope.

### <Condition 3: Spheroids consisting only of hepatocytes>

1.5 × 10⁵ human hepatocytes (PXB cells (registered trademark)) were suspended in an HCM containing 1 v/v% ECGS to a cell density of 1.5 × 10³ cells/200 µL to obtain a cell suspension. 200 µL of the cell suspension was seeded into a NucLon (registered trademark) 96-well plate (product number "174925", manufactured by Thermo Fisher). After performing CDFDA treatment and immunostaining in the same manner as in Condition 1 of Test Example 3, and the sample was evaluated using a confocal microscope.

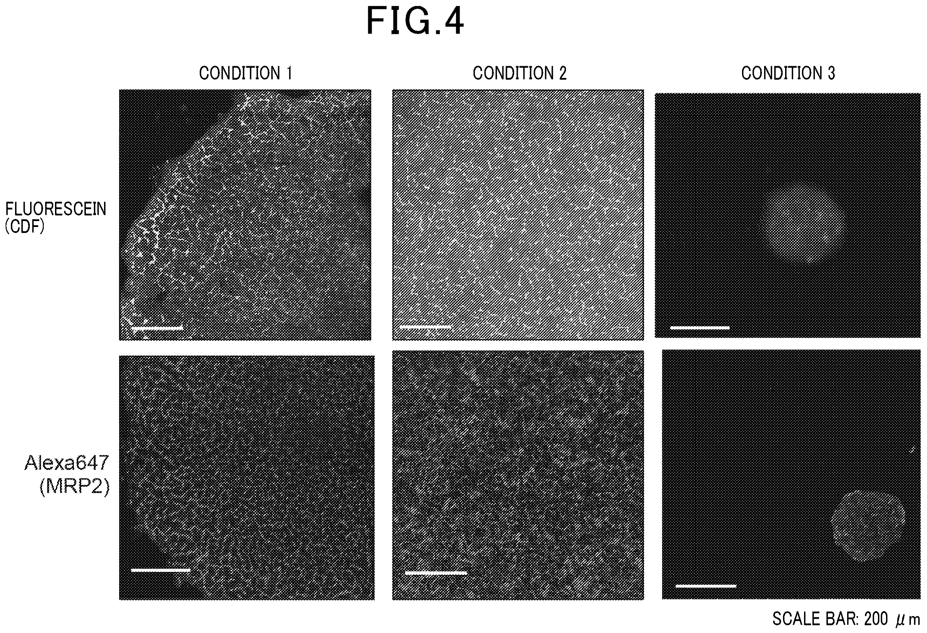

Fig. 4 is a photograph showing the results of observation using a confocal microscope. The fluorescein observation results indicate that localization of fluorescein in a linear or mesh-like form was observed in the three-dimensional hepatic tissue under Condition 1 and the sandwich-cultured tissue under Condition 2. Therefore, it was confirmed that CDFDA was taken up by hepatocytes, and its hydrolysate, CDF, was excreted into the bile canalicular structure, and that the bile canalicular structure was functioning normally. On the other hand, in the spheroids under Condition 3, no linear signal indicating the excretion of CDF into the bile canalicular structure was observed, suggesting that some of the functions of the hepatocytes were not functioning normally.

The MRP2 observation results shows that a certain level of expression occurred in the spheroids under Condition 3 as well. MRP2 is a transporter expressed on the bile canaliculi formed between normally differentiated and tightly adhering hepatocytes, and this transporter excretes CDF into the bile canaliculus structure. Therefore, it is considered that, although the spheroids under Condition 3 partially reproduced the structure, they did not reproduce the function, and form a tissue that is distinct from normal liver tissue.

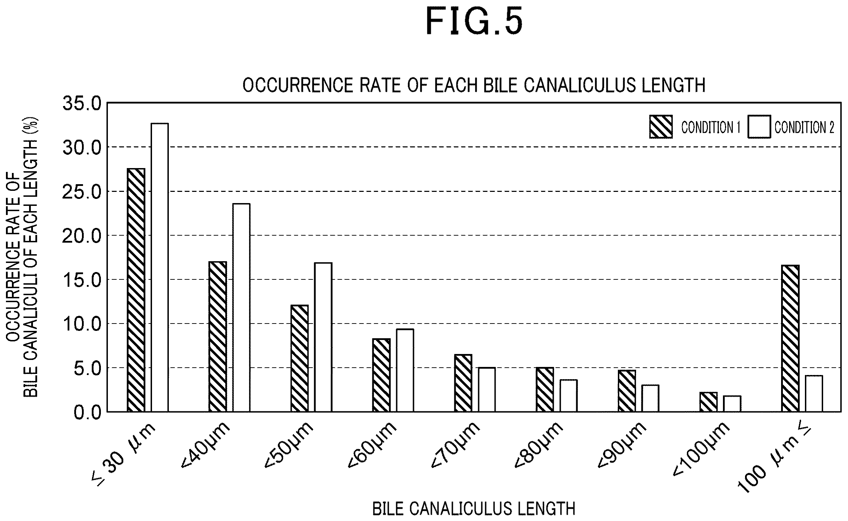

Fig. 5 is a graph showing the results of image analysis of the observed images in Fig. 4, comparing the occurrence rate of signals of each length obtained by measuring the length of each linear signal. In the sandwich-cultured under Condition 2, there were more short fragments in general, and the occurrence ratio of fragments exceeding 100 µm was 5% or less of the total, whereas in the three-dimensional hepatic tissue under Condition 1, the occurrence ratio of fragments exceeding 100 µm was 15% or more of the total.

### [Test Example 4: Production of three-dimensional hepatic tissue and toxicity evaluation 1 of cholestasis-inducing compounds]

### <Condition 1: Tissue consisting only of hepatocytes>

After producing three-dimensional hepatic tissue in the same manner as in Condition 1 of Test Example 2, on day 8 of culture, the medium was replaced with an HCM containing 1 v/v% ECGS, 10 µM cyclosporine A (product number "C2408", manufactured by Tokyo Chemical Industry Co., Ltd.), and 0.1% DMSO, and the tissue was incubated in an incubator at 37°C for 24 hours. At this time, an additional sample was prepared by replacing the medium with an HCM containing 1 v/v% ECGS and a bile acid cocktail having the composition shown in Table 5 along with cyclosporine A, and similarly incubated for 24 hours. In addition, as a comparative experiment, a sample was prepared by replacing the medium with a medium not containing cyclosporine A for each of the prepared samples. 100 µL of medium was collected from each sample, and LDH in the culture supernatant was quantified using Cytotoxicity LDH Assay Kit-WST (product number "CK12", manufactured by DOJINDO LABORATORIES). In addition, the remaining samples were subjected to an ATP assay using CellTiter Glo (registered trademark) 3D Viability Assay (product number "G9681", manufactured by Promega) to evaluate viability.

**[Table 5]**

| | Name | Concentration in medium [µM] |
|---|---|---|
| 1 | Chenodeoxycholic acid | 40.8 |
| 2 | Cholic acid | 24 |
| 3 | Deoxycholic acid | 88.08 |
| 4 | Glycochenodeoxycholic acid sodium salt | 205.2 |
| 5 | Glycocholic acid | 49.2 |
| 6 | Glycodeoxycholic acid | 45.6 5 |
| 7 | Lithocholic acid | 3.6 |
| 8 | Taurochenodeoxycholic acid | 25.2 |
| 9 | Taurocholic acid | 5.76 |
| 10 | Taurolithocholic acid | 10.44 |
| 11 | Taurolithodeoxycholic acid | 34.44 |
| 12 | Ursodeoxycholic acid | 13.2 |

### <Condition 2: Sandwich-cultured tissue consisting only of hepatocytes>

A sandwich-cultured tissue was produced in the same manner as in Condition 2 of Test Example 2. The tissue was exposed to cyclosporine A in the same manner as in Condition 1 of Test Example 4, and then the amount of LDH in the culture supernatant and the cell viability were evaluated.

### <Condition 3: Spheroids consisting only of hepatocytes>

Spheroids were produced in the same manner as in Condition 3 of Test Example 3. They were exposed to cyclosporine A in the same manner as in Condition 1 of Test Example 4, and then the amount of LDH in the culture supernatant and the cell viability were evaluated.

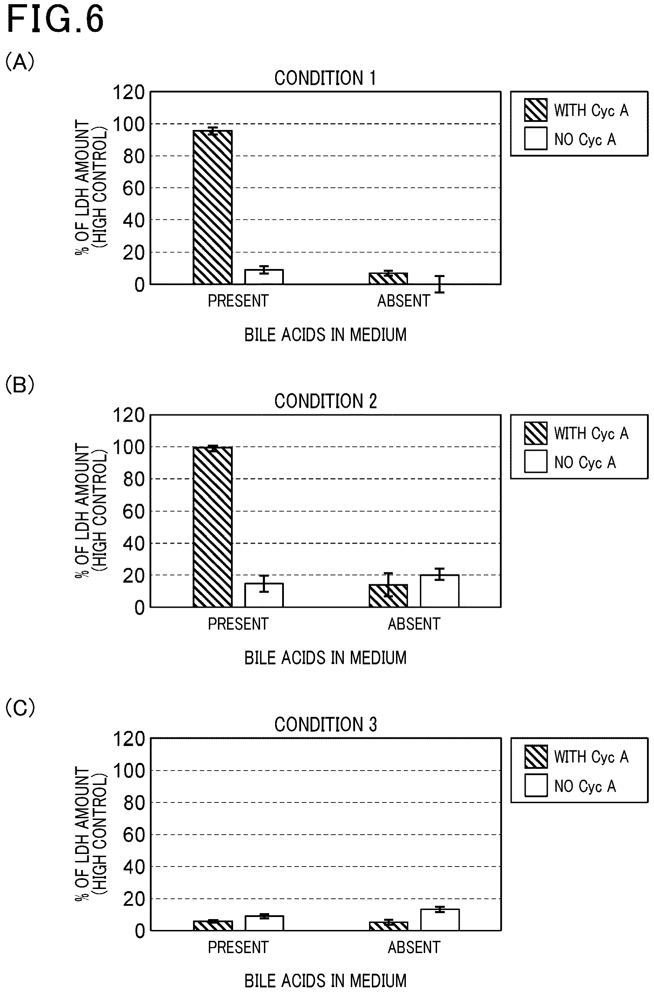

Fig. 6 is a graph showing the results of quantifying LDH in the culture supernatant. The vertical axis indicates the signal value obtained from each sample expressed as a ratio to a high control sample. The high control sample was obtained by preparing a separate control sample that was not exposed to bile acid and cyclosporine A, dissolving it immediately before evaluation, and evaluating the signal from the total LDH leaked therefrom. The three-dimensional hepatic tissue under Condition 1 and the sandwich-cultured tissue under Condition 2 showed very high values when they were treated with both bile acid and cyclosporine A, suggesting that the hepatocytes were severely damaged and the LDH inside leaked out. Cyclosporine A is a compound known to have the side effect of inhibiting the excretion of bile acids into the bile canaliculi. It is therefore considered that, when cyclosporine A and a large amount of bile acids were present, the former inhibited the excretion of bile acid into the bile canaliculi after they had been taken up by hepatocytes, resulting in the manifestation of toxicity. On the other hand, since no toxicity was observed in the spheroids under Condition 3, it is considered that they did not take up or excrete bile acids normally in the first place, and therefore the toxicity of cyclosporine A could not be evaluated. These results more strongly indicate the functional incompleteness of the spheroids under Condition 3, which was also suggested by the results of Test Example 3.

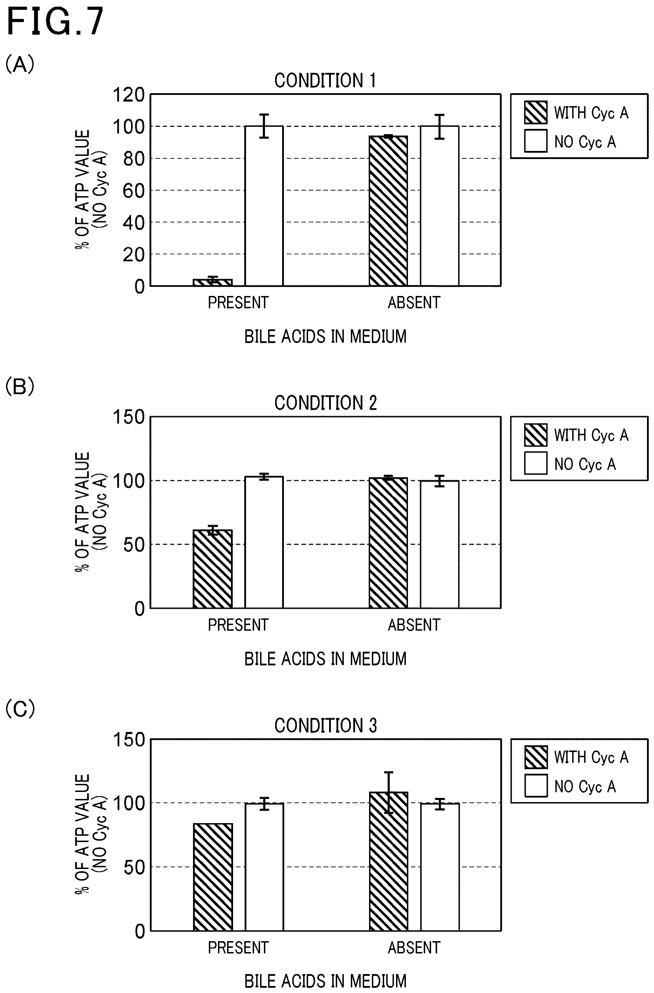

Fig. 7 is a graph showing the results of measuring the cell viability. The vertical axis indicates the relative ATP value of each sample, assuming that the ATP value of a sample not administered with cyclosporine A is 100. As shown in Fig. 7, generally the same tendency as in Fig. 6 was confirmed. Note that the ATP value of the sandwich-cultured tissue under Condition 2 treated with both bile acids and cyclosporine A was about half, which suggests that the three-dimensional hepatic tissue under Condition 1 may be able to evaluate cholestatic toxicity with higher sensitively (i.e., the original bile acid uptake and excretion functions are more intact).

### [Test Example 5: Production of three-dimensional hepatic tissue and toxicity evaluation 2 of cholestasis-inducing compounds]

### <Condition 1: Tissue consisting only of hepatocytes>

After producing three-dimensional hepatic tissue samples in the same manner as in Condition 1 of Test Example 2, on day 8 of culture, for each sample, the medium was replaced with an HCM containing 1 v/v% ECGS, one of the four compounds shown in Table 6, a bile acid cocktail having the composition shown in Table 5, and 0.1% DMSO, and the samples were incubated in an incubator at 37°C for 168 hours. In addition, as a comparative experiment, for each of the prepared samples, a sample was prepared by replacing the medium with a medium not containing the corresponding compound. Then, each sample was subjected to an ATP assay using CellTiter Glo (registered trademark) 3D Viability Assay (product number "G9681", manufactured by Promega) to evaluate viability.

**[Table 6]**

| Compound name | Exposure concentration |
|---|---|
| Cyclosporine A | 77 µM, 25 µM, 9 µM, 3 µM, or 1 µM |
| Atorvastatin | 6 µM, 2 µM, 0.6 µM, 0.2 µM, or 0.07 µM |
| Clomipramine | 79 µM, 27 µM, 9 µM, 3 µM, or 1 µM |
| Simvastatin | 239 µM, 80 µM, 27 µM, 9 µM, or 3 µM |

### <Condition 2: Spheroids consisting only of hepatocytes>

Spheroids were produced in the same manner as in Condition 3 of Test Example 3. They were exposed to each compound in the same manner as in Condition 1 of Test Example 5, and then the cell viability was evaluated.

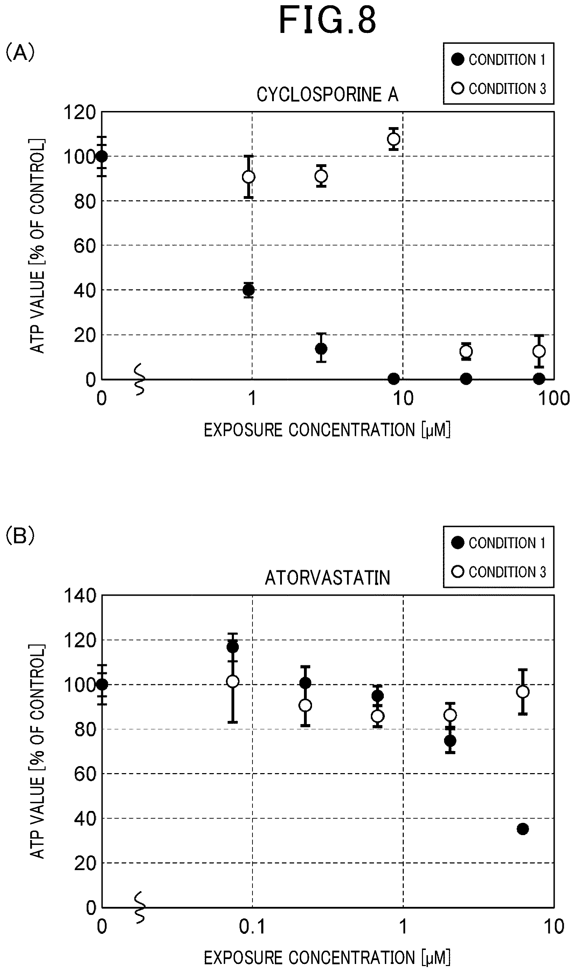

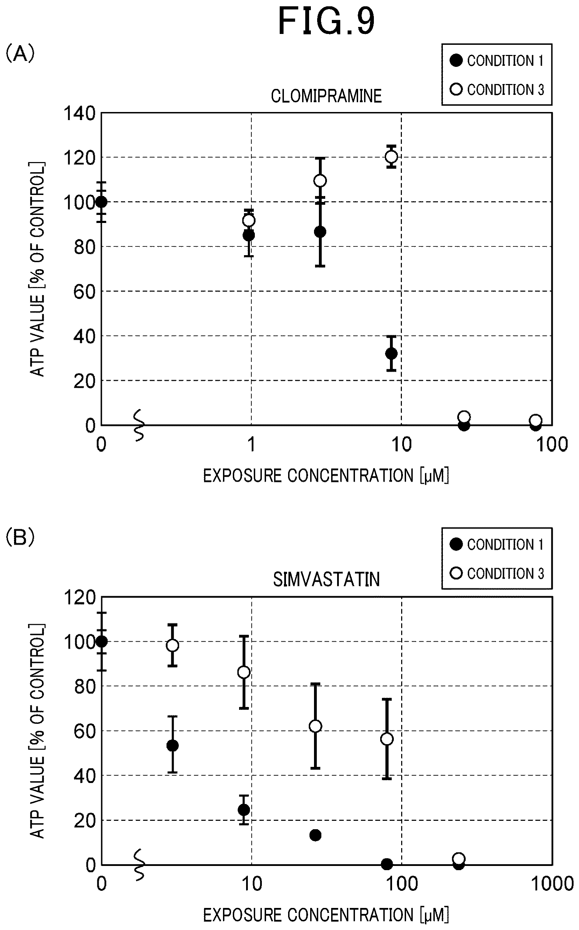

Fig. 8 is a graph showing the results of measuring the cell viability after exposure to cyclosporine A (Fig. 8(A)) and atorvastatin (Fig. 8(B)). Fig. 9 is a graph showing the results of measuring the cell viability after exposure to clomipramine (Fig. 9(A)) and simvastatin (Fig. 9(B)). In Figs. 8 and 9, the vertical axis indicates the relative ATP value for each tissue, assuming that the ATP value in the absence of exposure to the compounds is 100. These compounds are known to have side effects such as causing cholestasis in the liver of a living body and associated hepatotoxicity. It was found that for all of these four compounds, the toxicity manifested at a lower exposure concentration in the three-dimensional hepatic tissue under Condition 1, which is an example of the present invention. This result more strongly indicates the functional incompleteness of the spheroids under, which was also suggested by the results of Test Examples 3 and 4.

### [Test Example 6: Production of three-dimensional hepatic tissue and toxicity evaluation 3 of cholestasis-inducing compounds]

### <Condition 1: Tissue consisting only of hepatocytes>

After producing three-dimensional hepatic tissue in the same manner as in Condition 1 of Test Example 2, on day 8 of culture, the medium was replaced with an HCM containing 1 v/v% ECGS and each of the 16 hepatotoxicity-inducing compounds shown in Table 7, and the tissue was incubated in an incubator at 37°C for 168 hours. Then, each sample was subjected to an ATP assay using CellTiter Glo (registered trademark) 3D Viability Assay (product number "G9681", manufactured by Promega) to evaluate viability.

### <Condition 2: Spheroids consisting only of hepatocytes>

Spheroids were produced in the same manner as in Condition 3 of Test Example 3. They were exposed to each compound in the same manner as in Condition 1 of Test Example 6, and then the cell viability was evaluated.

Table 7 shows the IC₅₀ results calculated based on the cell viability measured after exposure to each compound at the corresponding concentration.

**[Table 7]**

| Drug name | IC₅₀ [µM] | | Compounds with 2-fold or greater difference in IC₅₀ value |
|---|---|---|---|
| | Condition 1 (present tissue) | Condition 2 (spheroid) | |
| Acetaminophen | 5513.0 | 4669.0 | |
| Amiodarone | 32.3 | 52.2 | |
| Diclofenac | 337.4 | 297.2 | |
| Nefazodone | 39.9 | 46.5 | |
| Perhexiline | 3.6 | 5.6 | |
| Tolcapone | 132.1 | 92.4 | |
| Troglitazone | 92.9 | 201.6 | Yes |
| Benzbromarone | 249.4 | 255.5 | |
| Carbamazepine | 842.6 | 978.5 | |
| Chlorpromazine | 26.6 | 25.0 | |
| Flutamide | 63.9 | 178.3 | Yes |
| Ketoconazole | 80.7 | 79.5 | |
| Lapatinib | 8.4 | 27.3 | Yes |
| Nimesulide | 323.2 | 683.3 | Yes |
| Tamoxifen | 6.3 | 6.4 | |
| Ticlopidine | 99.3 | 141.5 | |

Since these compounds are known to have side effects that cause hepatotoxicity in the liver of a living body through mechanisms other than cholestasis, co-exposure to bile acids and each compound was not performed in this test example. Of these 16 compounds, four compounds showed IC₅₀ values that were half or less in the three-dimensional hepatic tissue under Condition 1, which is an example of the present invention, indicating that their toxicity was manifested at lower exposure concentrations. On the other hand, none of the compounds showed an IC₅₀ value that was half or less under Condition 2. These results more strongly indicate the superiority of the three-dimensional hepatic tissue of the present invention over spheroids in terms of the accuracy of toxicity prediction.

### [Test Example 7: Confirmation of the effect of presence of FBS in culture medium]

### <Condition 1: Culture in FBS-free medium>

Three-dimensional hepatic tissue was produced in the same manner as in Condition 1 of Test Example 2. Then, it was cultured in a normal HCM not containing FBS, and the medium was changed. On day 8 of culture, CDFDA treatment was performed in the same manner as in Condition 1 of Test Example 3, and the tissue was evaluated using a confocal microscope.

### <Condition 2: Culture in FBS-containing medium>

Three-dimensional hepatic tissue was produced in the same manner as in Condition 1 of Test Example 2. Then, it was cultured in an HCM containing 5 v/v% FBS, and the medium was changed. On day 8 of culture, CDFDA treatment was performed in the same manner as in Condition 1 of Test Example 3, and the tissue was evaluated using a confocal microscope.

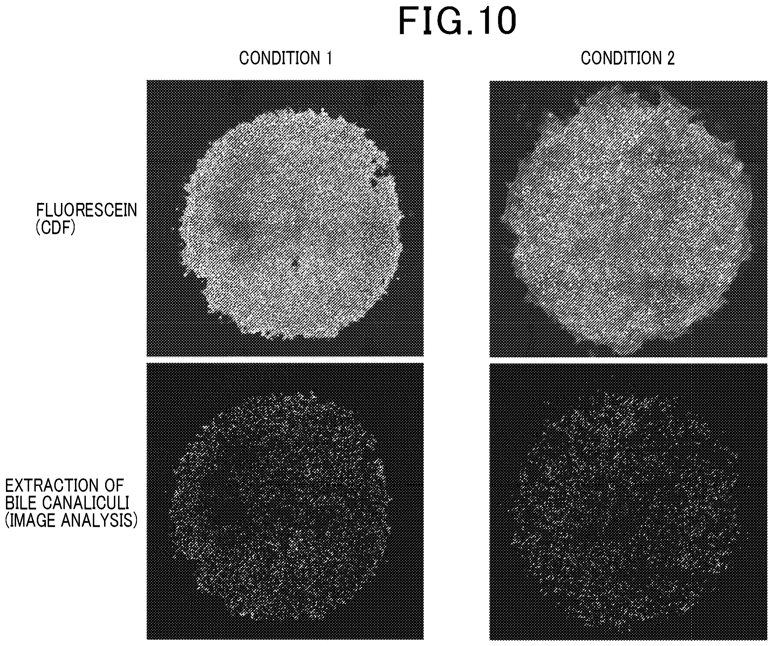

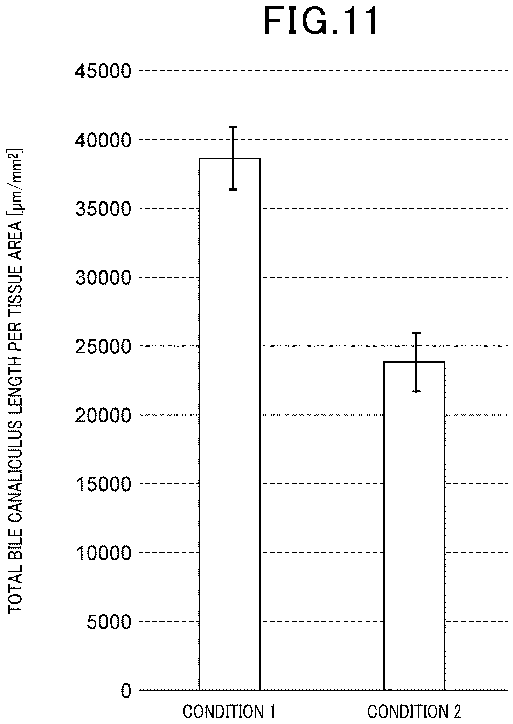

Fig. 10 is a photograph showing the results of observation using a confocal microscope. The fluorescein observation results in Fig. 10 show that localization of fluorescein in a linear or mesh-like form was observed in the three-dimensional hepatic tissue under Condition 1. This confirms that CDFDA was taken up by hepatocytes and its hydrolysate, CDF, was excreted into the bile canalicular structure, and that the bile canalicular structure was functioning normally. On the other hand, under Condition 2, the linear signal indicating the excretion of CDF into the bile canalicular structure was significantly less than under Condition 1, suggesting that the performance expected from liver tissue was not provided. Fig. 11 is a graph showing the results of image analysis of the observed images in Fig. 10, comparing the total bile canaliculus length per tissue area obtained by measuring the length of each linear signal. The three-dimensional hepatic tissue under Condition 1 had a longer total bile canaliculus length per tissue area than the three-dimensional hepatic tissue under Condition 2.

FBS is well known as a commonly used supplement in cell culture, and in the culture of hepatocytes, a medium containing FBS is commonly used as a hepatocyte culture medium in conventional culture systems. During exposure to drugs, there are cases where culture media containing FBS are not used due to concerns about the effects of drug's binding to proteins in the FBS. However, based on conventional technical common sense, it is difficult to imagine that the presence of FBS would in fact inhibit the formation of bile canaliculi in hepatocytes. These test results suggest the very unique properties of the three-dimensional hepatic tissue of the present invention, and also suggest that it can maintain high performance without using FBS, which is difficult to chemically define and has large lot-to-lot variation. In other words, the potential undesired effects of adding FBS, such as the drug's binding to proteins, can be avoided, and toxicity evaluation through repeated administration over a long period of time can be performed.

### [Test Example 8: Monitoring of albumin secretion]

Three-dimensional hepatic tissue was produced in the same manner as in Condition 1 of Test Example 2. The tissue was incubated in an incubator at 37°C for 28 days, changing the medium periodically using an HCM containing 1 v/v% ECGS. During the culture period, culture supernatant was collected on days 7, 10, 14, 17, 21, 24, and 28 of culture, and the amount of albumin in each supernatant sample was evaluated using a Human Albumin ELISA Quantitation Set (product number "E80-129", manufactured by Bethyl Laboratories). The medium was changed the day before collecting the supernatant, and the secreted albumin was allowed to accumulate for 24 hours before collection and evaluation.

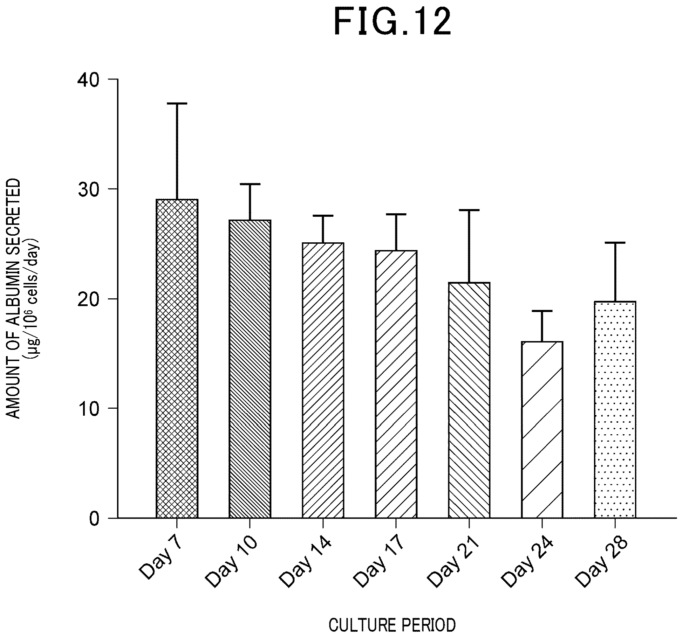

Fig. 12 is a graph showing the results of evaluation of the amount of albumin secreted. This three-dimensional hepatic tissue did not show a significant decrease in the amount of albumin secreted in an FBS-free culture medium environment up to day 28, suggesting that long-term culture is possible.

### [Test Example 9: Freezing test]

As in Condition 1 of Test Example 2, 2 µL of an HCM containing 5 mg/mL fibrinogen and 1 v/v% ECGS was added to a 48-well microplate (product number "3830-048", manufactured by AGC Techno Glass) to form a droplet. The cell suspension was added to the interior of each droplet, and the 48-well microplate was then left to stand in an incubator for 40 minutes to form a fibrin gel in which the human hepatocytes were embedded. 300 µL of cryopreservation medium (CELLBANKER (registered trademark) 1 plus (product number "11913", manufactured by ZENOGEN PHARMA)) was added to the wells, and the plate was placed in an 80°C freezer and stored frozen for one week. After one week, the plate was taken out of the freezer, 900 µL of culture medium heated to 37°C was added, and the plate was left to stand in an incubator for 20 minutes to thaw the cryopreservation medium. The medium was changed and the cryopreservation medium was removed immediately after that. Then, the cells were cultured for 7 days while changing the medium at appropriate times to produce three-dimensional hepatic tissue. On the 7th day after thawing, the viability of the cells in the gel was evaluated by treating the tissue with Calcein-AM included in the Live/Dead cell staining kit II (product number "PK-CA707-30002", manufactured by Promocell).

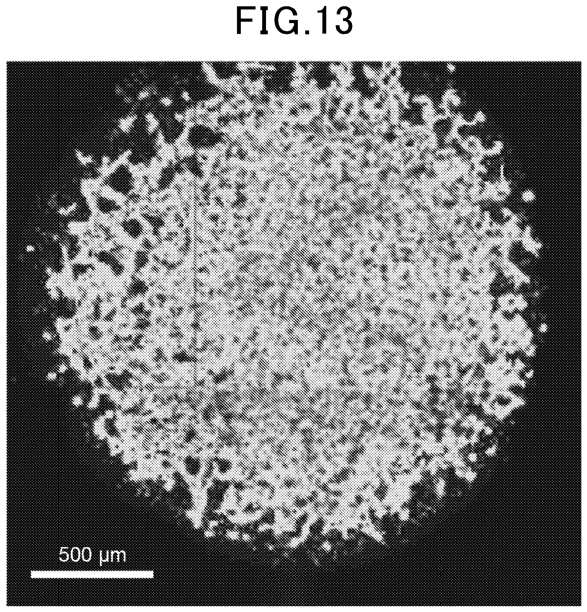

Fig. 13 shows an image of the tissue on the 7th day after thawing in which viable cells are stained with Calcein-AM. It was confirmed that the tissue produced in this example was able to continue to be cultured with the cells embedded in the gel and without the gel being damaged even when it was frozen and thawed immediately after gel formation, and that intercellular adhesion advanced and organization was promoted.

When freezing cells, freezing and thawing them when intercellular adhesion is in an advanced state (such as in a spheroid state) can significantly impair cell function. Therefore, primary hepatocytes are usually frozen by preparing a suspension in which the cells are monodispersed. However, if the final intended use of the cells is for three-dimensional culture, using cells frozen in a suspension will require additional work to produce tissue after thawing, making the process complicated. In the case of a tissue in which the cell structure of the present invention is embedded in a hydrogel, even if the tissue is frozen immediately after seeding when intercellular adhesion has not yet advanced, since the cells are embedded in the gel, the cells and the gel itself will not be scattered after freezing and thawing. Therefore, the tissue can be produced by continuing the culture and promoting organization. Since the tissue can be frozen and stored, the present example provides advantages such as making it possible to prepare a large amount of tissue at once and then store it, and facilitating control of the transport conditions during tissue transport, and demonstrates the convenience of the present invention.

## Claims

1. A three-dimensional hepatic tissue comprising a cell structure containing at least hepatocytes, and a hydrogel, wherein
the cell structure does not contain hepatic stellate cells, and
the cell structure is embedded in the hydrogel.

2. The three-dimensional hepatic tissue according to claim 1, wherein
an exterior of the cell structure is covered with the hydrogel, and no hydrogel is present in intercellular spaces inside the cell structure.

3. The three-dimensional hepatic tissue according to claim 1 or 2, wherein
the cell structure includes bile canaliculi.

4. The three-dimensional hepatic tissue according to claim 1 or 2, wherein
expression levels of CYP3A4 gene and CYP2C9 gene are maintained.

5. The three-dimensional hepatic tissue according to claim 1 or 2, wherein
the hydrogel is a fibrin gel.

6. The three-dimensional hepatic tissue according to claim 1 or 2, further comprising an extracellular matrix component.

7. The three-dimensional hepatic tissue according to claim 6, wherein
the extracellular matrix component is a collagen component.

8. The three-dimensional hepatic tissue according to claim 1 or 2, further comprising a polyelectrolyte.

9. The three-dimensional hepatic tissue according to claim 8, wherein
the polyelectrolyte is heparin.

10. A method of manufacturing three-dimensional hepatic tissue, the method comprising:
causing a composition containing cells and a hydrogel-forming substance to gel to embed the cells in the hydrogel; and
culturing the cells embedded in the hydrogel, wherein
the cells contain hepatocytes and no hepatic stellate cells.

11. The manufacturing method according to claim 10, wherein
the composition further contains an extracellular matrix component.

12. The manufacturing method according to claim 11, wherein
the extracellular matrix component is a collagen component.

13. The manufacturing method according to claim 10 or 11, wherein
the composition further contains a polyelectrolyte.

14. The manufacturing method according to claim 13, wherein
the polyelectrolyte is heparin.

15. The manufacturing method according to claim 10 or 11, wherein
the hydrogel-forming substance includes fibrinogen and thrombin.
